(19) Europäisches Patentamt
European Patent Office
Office européen des brevets

(11) Veröffentlichungsnummer: **0 369 335 B1**

# (12) EUROPÄISCHE PATENTSCHRIFT

(45) Veröffentlichungstag der Patentschrift: **23.02.94**

(21) Anmeldenummer: **89120843.1**

(22) Anmeldetag: **10.11.89**

(51) Int. Cl.[5]: **C07C 211/38**, A01N 33/08, A01N 33/04, C07C 217/08, C07C 217/46, C07C 217/58, C07C 217/48, C07C 217/60, C07D 207/06, C07D 211/70, C07C 215/44

## (54) Neue 2-Aminodekalin-Derivate und deren Verwendung.

(30) Priorität: **15.11.88 DE 3838631**

(43) Veröffentlichungstag der Anmeldung: **23.05.90 Patentblatt 90/21**

(45) Bekanntmachung des Hinweises auf die Patenterteilung: **23.02.94 Patentblatt 94/08**

(84) Benannte Vertragsstaaten: **AT BE CH DE ES FR GB GR IT LI NL SE**

(56) Entgegenhaltungen:
**EP-A- 0 254 150**
**EP-A- 0 309 913**
**EP-A- 0 309 914**
**EP-A- 0 355 544**

**SYNTHESIS, November 1987, Seiten 1005-1007, Stuttgart, DE; H.-J. SCHÄFER et al.: "Reductive amination of ketones and aldehydes at the mercury-cathode"**

(73) Patentinhaber: **BASF Aktiengesellschaft
Carl-Bosch-Strasse 38
D-67063 Ludwigshafen(DE)**

(72) Erfinder: **Zipplies, Matthias, Dr.
Kastanienweg 1
D-6945 Hirschberg(DE)**
Erfinder: **Zipperer, Bernhard, Dr.
Am Herrgottsacker 6
D-6716 Dirmstein(DE)**
Erfinder: **Sauter, Hubert, Dr.
Neckarpromenade 20
D-6800 Mannheim 1(DE)**
Erfinder: **Goetz, Norbert, Dr.
Schoefferstrasse 25
D-6520 Worms 1(DE)**
Erfinder: **Roehl, Franz, Dr.
Karl-Otto-Braun-Strasse 8
D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

## Beschreibung

Die vorliegende Erfindung betrifft N-substiuierte 2-Amino-dekalin-Derivate, Verfahren Zu deren Herstellung, deren Verwendung als Fungizide, fungizide Mischungen sowie Verfahren zur Bekämpfung von Pilzen mit diesen Wirkstoffen.

Die Verbindung 2-Amino-dekalin ist bekannt (Leroux, Comptes rendus de l'Academie de Sciences 141, 47; Leroux, Annales de Chimie et de Physique 21, 533). Die vier möglichen Diastereomeren von 2-Amino-dekalin sind in der Literatur ebenfalls beschrieben worden (Hückel, Liebigs Ann. der Chemie 533, 14 (1938). Dauben et al., J. Amer. Chem. Soc. 76, 4420 (1954)). Es sind ferner die Infrarotspektren von N-Methyl- und N,N-Dimethyl-dekalylamin-Diastereomeren beschrieben worden (Feltkamp, Liebigs Ann. Chem. 683, 49 - 54 (1965)). Die Synthese von N-Ethyl-dekalylamin ist in der Literatur beschrieben worden (T. Pienemann et al., Synthesis 1987, 1005). In EP-A-309 913, 309 914 und 355 544 werden 2-Aminodekalinderivate mit fungizider Wirksamkeit beschrieben.

In EP 254 150 wird das Dekalyl-alkyl-morpholin-Derivat VII als Verbindung mit fungizider Wirkung beschrieben.

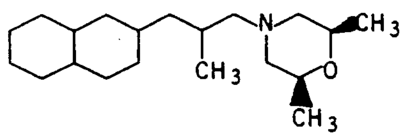

VII

Es wurde gefunden, daß 2-Amino-dekalin-Derivate der Formel I

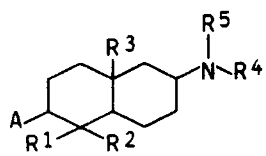

I

in der die Substituenten $R^1$ bis $R^5$ und A die folgende Bedeutung haben:

$R^1$, $R^2$, $R^3$ sind gleich oder verschieden und bedeuten H oder Methyl,

A = OH, O-$C_1$-$C_4$-Alkylcarbonyl, O-Benzoyl,

$R^4$ = H, $C_1$-$C_4$-Alkyl, $C_2$-$C_4$-Alkenyl oder $C_3$-$C_4$-Alkinyl, Cyclopropyl,

$R^5$ = $C_3$-$C_{18}$-Alkyl, $C_3$-$C_{18}$-Alkenyl oder $C_3$-$C_{18}$-Alkinyl, welches gegebenenfalls einfach bis dreifach durch $C_1$-$C_8$-Alkoxy substituiert ist,

$C_5$-$C_{12}$-Cycloalkyl oder $C_5$-$C_{12}$-Cycloalkenyl, welches gegebenenfalls einfach bis dreifach durch $C_3$-$C_8$-Alkyl, $C_3$-$C_8$-Alkenyl oder $C_3$-$C_8$-Alkoxy substituiert ist,

oder $R^5$ bedeutet die Reste

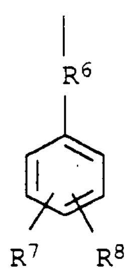 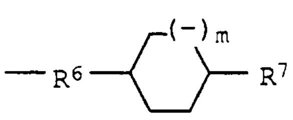 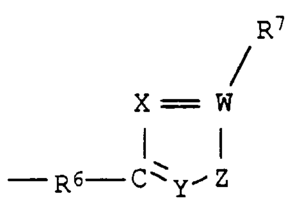 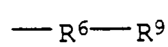

a b c d

worin die Reste $R^6$ bis $R^9$, die Buchstaben W, X, Y, Z sowie der Index m die folgende Bedeutung haben:

$R^6$ = $C_1$-$C_5$-Alkylen oder $C_2$-$C_5$-Alkenylen,

$R^7$ = $C_3$-$C_8$-Alkyl, $C_3$-$C_8$-Alkenyl, $C_3$-$C_8$-Alkoxy, $C_1$-$C_3$-Alkoxy-$C_2$-$C_5$-Alkyl, $C_3$-$C_5$-Alkylthio oder Trimethylsilyl,

$R^8$ = H, $CH_3$,
$R^9$ = gegebenenfalls mit 2 bis 3 Methylgruppen substituiertes $C_6$-$C_{10}$-Bicycloalkyl oder $C_6$-$C_{10}$-Bicycloalkenyl,
W, X, Y, Z = bedeuten die Ringatome in einem aromatischen 5-Ring Heterocyclus, und zwar 1 bis 2 Reste aus der Gruppe O, S, N, NH, N-$R^7$ und 2 bis 3 Reste CH, C-$R^7$,
m bedeuten 0, 1, 2; so daß der Rest 5b einen Cyclohexyl-, Cycloheptyl- oder Cyclopentylrest enthält,
Wenn A O-$C_1$-$C_4$-Alkylcarbonyl oder O-Benzoyl bedeutet, können $R^4$ und $R^5$ auch gemeinsam mit dem Stickstoff, dessen Substituenten sie sind, einen 5- oder 6-gliedrigen Ring e oder f bedeuten,

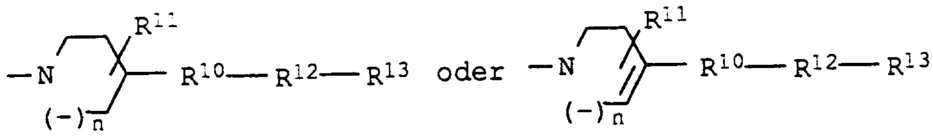

e oder f

wobei die Reste $R^{10}$ bis $R^{13}$ sowie der Index n die folgende Bedeutung haben:
$R^{10}$ = H oder $(CH_2)_q$ mit q = 0, 1, 2, 3, 4 oder Methyl, Ethyl oder verzweigtes $C_3$-$C_8$-Alkyl,
$R^{11}$ = H, $C_1$-$C_5$-Alkyl,
$R^{12}$, wenn $R^{10}$ verschieden von H ist, bedeutet $R^{12}$ H oder Phenyl,
$R^{13}$, für den Fall, daß $R^{12}$ Phenyl bedeutet, bedeutet $R^{13}$ $C_3$-$C_6$-Alkyl,
n = 0, 1, so daß der Rest 5e oder 5f einen 6-Ring oder 5-Ring Heterocyclus enthält,
zusätzlich bedeutet
A = H, O-$CH_3$, in diesem Falle bedeutet
$R^4$ = H, $C_1$-$C_4$-Alkyl, $C_2$-$C_4$-Alkenyl oder $C_3$-$C_4$-Alkinyl, Cyclopropyl,
$R^5$ bedeutet die Reste c und d

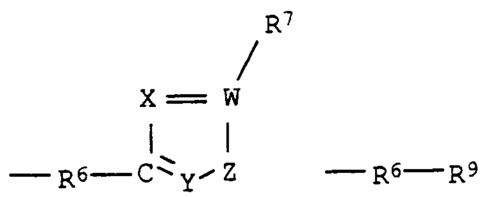

c d

worin die Reste $R^6$ bis $R^9$ und die Buchstaben W, X, Y, Z die folgende Bedeutung haben:
$R^6$ = $C_1$-$C_5$-Alkylen oder $C_2$-$C_5$-Alkenylen,
$R^7$ = $C_3$-$C_8$-Alkyl, $C_3$-$C_8$-Alkenyl, $C_3$-$C_8$-Alkoxy, $C_1$-$C_3$-Alkoxy-$C_2$-$C_5$-Alkyl, $C_3$-$C_5$-Alkylthio oder Trimethylsilyl,
$R^9$ = gegebenenfalls mit 2 bis 3 Methylgruppen substituiertes $C_6$-$C_{10}$-Bicycloalkyl oder $C_6$-$C_{10}$-Bicycloalkenyl,
W, X, Y, Z = bedeuten die Ringatome in einem aromatischen 5-Ring Heterocyclus, und zwar 1 bis 2 Reste aus der Gruppe O, S, N, NH, N-$R^7$ und 2 bis 3 Reste CH, C-$R^7$,
Wenn A H, OH oder O-$CH_3$ bedeutet, können $R^4$ und $R^5$ auch gemeinsam mit dem Stickstoff, dessen Substituenten sie sind, einen 5- oder 6-gliedrigen Ring e bedeuten,

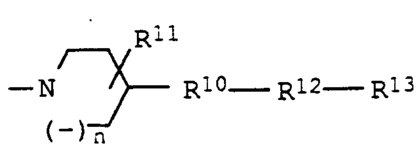

e

wobei die Reste $R^{10}$ bis $R^{13}$ sowie der Index n die folgende Bedeutung haben:
n = 1
$R^{10}$ = $(CH_2)_q$ mit q = 0, 1, 2, 3, 4 oder Ethyl oder verzweigtes $C_3$-$C_8$-Alkyl,
$R^{11}$ = H, $C_1$-$C_5$-Alkyl,
$R^{12}$, wenn $R^{10}$ den Rest $(CH_2)_q$ mit q = 0, 1, 2, 3, 4 bedeutet, bedeutet $R^{12}$ Phenyl und,
$R^{13}$ bedeutet $C_3$-$C_6$-Alkyl,
zusätzlich bedeutet n = 0, in diesem Fall bedeutet $R^{10}$ = $(CH_2)_q$ mit q = 1, 2, 3, 4 oder Ethyl oder verzweigtes $C_3$-$C_8$-Alkyl,
$R^{11}$ = H, $C_1$-$C_5$-Alkyl,
$R^{12}$, wenn $R^{10}$ den Rest $(CH_2)_q$ mit q = 1, 2, 3, 4 bedeutet, bedeutet $R^{12}$ Phenyl und,
$R^{13}$ bedeutet $C_3$-$C_6$-Alkyl,
$R^4$ und $R^5$ können auch gemeinsam mit dem Stickstoff, dessen Substituenten sie sind, einen 5- oder 6-gliedrigen Ring f bedeuten,

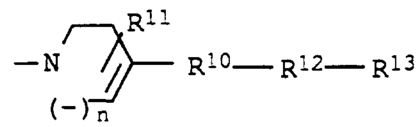

f

wobei die Reste $R^{10}$ bis $R^{13}$ sowie der Index n die folgende Bedeutung haben:
$R^{10}$ = $(CH_2)_q$ mit q = 0, 1, 2, 3, 4 oder Methyl, Ethyl oder verzweigtes $C_3$-$C_8$-Alkyl,
$R^{11}$ = H, $C_1$-$C_5$-Alkyl,
$R^{12}$, wenn $R^{10}$ den Rest $(CH_2)_q$ mit q = 0, 1, 2, 3, 4 bedeutet, bedeutet $R^{12}$ Phenyl, und
$R^{13}$ bedeutet $C_3$-$C_6$-Alkyl,
n = 0, 1, so daß der Rest 5e oder 5f einen 6-Ring oder 5-Ring Heterocyclus enthält,
sowie deren pflanzenverträgliche Salze und N-Oxide
bei guter Pflanzenverträglichkeit stark fungizid wirken.

Von der vorliegenden Erfindung werden sowohl die trans- als auch die cis-Isomere der Formel I und ihre Mischungen umfaßt. Diese Verbindungen können als Fungizide verwendet werden.

Bevorzugt werden die Diastomere der Formel II

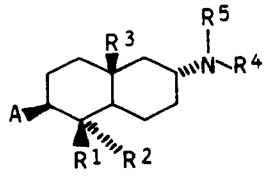

II

bei denen es sich um die trans-Dekalin-Derivate mit equatorialer Stellung sowohl der Aminofunktion als auch - soweit vorhanden - des Restes A handelt.

Als Wirkstoffe kommen aus praktischen Gründen auch die Salze der erfindungsgemäßen Dekalylamine in Betracht. Darunter sind die Salze der Amine mit beliebigen anorganischen oder organischen Säuren bzw. Protonen-aciden Verbindungen im weitesten Sinne zu verstehen, z. B. mit Chlorwasserstoff, Bromwasserstoff, Jodwasserstoff, Essigsäure, Propionsäure, Palmitinsäure, Stearinsäure, Perfluorheptansäure, Oxalsäure, Malonsäure, Benzoesäure, Äpfelsäure, Dodecylsulfonsäure, alpha-Naphthalinsulfonsäure, $\beta$-Naphthalinsäure, 2,6-Dichlor-isonicotin-säure, Saccharin, Salicylsäure, Glycerin-2-phosphorsäure, Methansulfonsäure, Dodecylbenzolsulfonsäure, p-Toluolsulfonsäure.

$R^4$ bedeutet beispielsweise Wasserstoff, Methyl, Ethyl, Propyl, Isopropyl, n-Butyl, Iso-butyl, sek.-Butyl, Allyl, Propargyl oder Cyclopropyl.

$R^5$ bedeutet beispielsweise verzweigtes oder unverzweigtes $C_3$-$C_{18}$-Alkyl, insbesondere $C_8$-$C_{16}$-Alkyl, beispielsweise n-Propyl, n-Hexyl, n-Heptyl, 4,4-Dimethylpentyl, n-Octyl, 6,6-Dimethyl-hept-2-yl, n-Nonyl, 3,5,5-Trimethylhexyl, n-Decyl, 3,7-Dimethyloct-2-yl, n-Undecyl, n-Dodecyl, Tridecyl, Hexadecyl, Octadecyl, 3,7-Dimethyl-octyl, 6,10-Dimethyl-undec-2-yl, 2,3,5,5-Tetramethyl-hexyl, 2,4,5,7,7-Pentamethyl-octyl, oder $C_3$-$C_{18}$-Alkenyl, wie beispielsweise $C_8$-$C_{12}$-Alkenyl, z.B. 3,7-Dimethyl-oct-6-en-yl,

oder $C_3$-$C_{18}$-Alkinyl, wie beispielsweise $C_6$-$C_{10}$-Alkinyl, z.B. 4,4-Dimethyl-pent-2-in-yl, oder mit verzweigtem oder unverzweigtem $C_1$-$C_8$-Alkoxy einfach bis dreifach substituiertes $C_3$-$C_{18}$-Alkyl, $C_8$-$C_{16}$-Alkyl, $C_3$-$C_{18}$-Alkenyl oder $C_3$-$C_{18}$-Alkinyl wie beispielsweise 4-Methoxy-4-methyl-pentyl oder 4-Methoxy-4-methyl-pent-2-in-yl, 2-tert.-Butoxy-propyl, 2-tert.-Amyloxy-propyl, 3-tert.-Butoxy-2-methylbutyl, 3-tert.-Butoxy-propyl.

$R^5$ kann auch $C_5$-$C_{12}$-Cycloalkyl oder $C_5$-$C_{12}$-Cycloalkenyl bedeuten, welches einfach bis dreifach gegebenenfalls mit verzweigtem oder unverzweigtem $C_3$-$C_8$-Alkyl, $C_3$-$C_8$-Alkenyl oder $C_3$-$C_8$-Alkoxy substituiert sein kann, wie beispielsweise Cyclohexyl, 4-tert.-Butyl-cyclohexyl, 4-Isopropyl-cyclohexyl, 4-(2,4,4-Trimethyl-pent-2-yl)cyclohexyl, 4-tert-Butoxy-cyclohexyl, 4-tert.-Butyl-cyclohexenyl;

$R^5$ kann auch für den Rest a

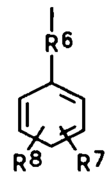

a

stehen, worin

$R^6$ gegebenenfalls verzweigtes oder unverzweigtes $C_1$-$C_5$-Alkylen oder $C_2$-$C_5$-Alkenylen, z.B. Methylen,

$R^7$ verzweigtes oder unverzweigtes $C_3$-$C_8$-Alkyl, $C_3$-$C_8$-Alkenyl, $C_3$-$C_8$-Alkoxy, $C_1$-$C_3$-Alkoxy-$C_2$-$C_5$-Alkyl, $C_3$-$C_5$-Alkylthio oder den Trimethylsilylrest, z.B. tert.Butyl,

$R^8$ Wasserstoff oder Methyl bedeuten.

Beispiele für erfindungsgemäße Reste der Struktur a sind z.B. 4-tert-Butyl-benzyl, 4-tert-Butoxy-benzyl, 4-Trimethylsilyl-benzyl, 4-tert-Butylthio-benzyl, 4-tert-Butyl-phenylethyl, 4-tert-Butyl-phenylpropyl, 1(4-tert-Butyl-phenyl)-2-methyl-propyl, 1(4-tert-Butyl-2-methyl-phenyl)-2-methyl-propyl, 4(2,3-Dimethyl-but-2-yl)-benzyl, 4(2-Methyl-but-2-yl)benzyl, 4(2,4,4-Trimethyl-pent-2-yl)benzyl, 4-tert Butoxy-phenylethyl, 4(2-Methoxy-prop-2-yl)benzyl,

$R^5$ kann auch für den Rest b stehen,

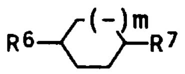

b

worin $R^6$ und $R^7$ dieselbe Bedeutungen wie in oben haben und m = 0,1,2 bedeutet, so daß der Rest 5b einen Cyclohexyl-, Cycloheptyl- oder Cyclopentylrest enthält,

Beispiele für Reste b sind:

4-tert.-Butyl-cyclohexyl-methyl,

4-tert.-Butoxy-cyclohexyl-methyl,

1(4-tert.-Butyl-cyclohexyl)-2-methyl-propyl.

$R^5$ kann auch für den Rest c stehen

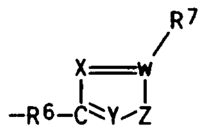

c,

worin $R^6$ und $R^7$ dieselbe Bedeutung wie oben haben und W,X,Y,Z bedeuten die Ringatome in einem aromatischen 5-Ring Heterocyclus und zwar 1 bis 2 Reste aus der Gruppe O, S, N, NH, N-$R^7$ und 2 bis 3 Reste CH, C-$R^7$.

Beispiele für erfindungsgemäße Reste c sind:

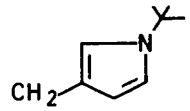

3-(N-tert.-Butyl)pyrryl-methyl,

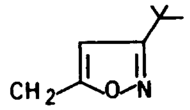

5-(3-tert.-Butyl)isoxazolyl-methyl,

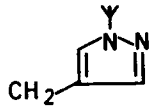

4-(N-tert.-Butyl)pyrazolyl-methyl.

$R^5$ kann weiterhin für den Rest d stehen

$-R^6-R^9$ d

worin $R^6$ dieselbe Bedeutung wie oben besitzt, $R^9$ hingegen einen gegebenenfalls mit 1 - 3 Methylgruppen substituierten $C_6$-$C_{10}$-Bicycloalkyl- oder $C_6$-$C_{10}$-Bicycloalkenyl-Rest bedeutet.

Beispiele für den Rest d sind:

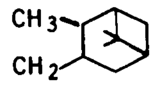

3(2,6,6-Trimethyl[3.1.1]bicycloheptyl)methyl,

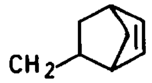

5-([2.2.1]Bicyclohept-2-en-yl)-methyl

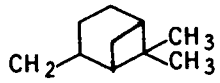

2(6,6-Dimethyl[3.1.1]bicycloheptyl)methyl

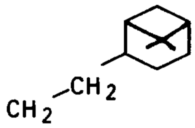

2(6,6-Dimethyl[3.1.1]bicycloheptyl)ethyl.

$R^4$ und $R^5$ können aber auch gemeinsam mit dem Stickstoffatom, dessen Substituenten sie sind, einen 5- oder 6-gliedrigen Ring (Reste e und f) bedeuten, wobei

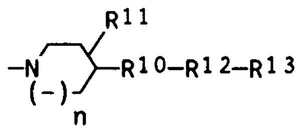

e

oder

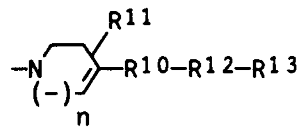

f

$R^{10}$ Wasserstoff oder die Gruppe $(CH_2)_q$ mit q = 0,1,2,3,4 oder Methyl, Ethyl oder verzweigtes $C_3$-$C_8$-Alkyl bedeutet,
$R^{11}$ Wasserstoff oder verzweigtes oder unverzweigtes $C_1$-$C_5$-Alkyl bedeutet,
$R^{12}$, für den Fall, daß $R^{10}$ verschieden von H ist, Wasserstoff oder den Phenyl-Rest bedeutet,
$R^{13}$, für den Fall, daß $R^{12}$ Phenyl bedeutet, $R^{13}$ verzweigtes oder unverzweigtes $C_3$-$C_6$-Alkyl bedeutet und n den Wert 0 oder 1 hat, so daß der Rest 5e oder 5f einen 6-Ring oder 5-Ring Heterocyclus enthält.

Beispiele für erfindungsgemäße Reste e oder f sind:
4-Methyl-piperidinyl, 3,3-Dimethyl-piperidinyl,
3-Ethyl-4-propyl-piperidinyl, 4-Phenyl-piperidinyl,
4-Benzyl-piperidinyl, 4-(3-Phenylpropyl)-piperidinyl,
4[3(4-tert-Butyl-phenyl)propyl]piperidinyl, 4(4 tert-Butyl-phenyl)piperidinyl, 4(4-tert-Butyl-benzyl)piperidinyl,
3(1,3,3-Trimethyl-butyl)piperidinyl,
2-Methyl-5(1,5-Dimethyl-hexyl)piperidinyl,
4(4-tert-Butylphenyl)-piperid-3-en-yl,
3-(1,5-Dimethyl-hexyl)pyrrolidinyl,
4(3,3-Dimethyl-butyl)piperid-3-en-yl,
4(3,3-Dimethyl-butyl)piperidinyl, 4-tert-Butyl-piperidinyl,
3(4-tert-Butyl-phenyl)pyrrolidinyl.

Die neuen N-substituierten 2-Aminodekaline und deren pflanzenphysiologisch verträglichen Salze enthalten chirale Zentren. Sie werden im allgemeinen als Racemate oder gegebenenfalls als Diastereomerengemische erhalten.

Diastereomerenreine Verbindungen lassen sich bei einigen der neuen Verbindungen beispielsweise durch Destillation, Säulenchromatographie oder aufgrund von Löslichkeitsunterschieden isolieren. Enantiomerenreine Verbindungen lassen sich z. B. durch Racematspaltung mit einem chiralen Hilfsreagenz nach bekannten Methoden beispielsweise über diastereomere Salze erhalten. Für die Anwendung der neuen 2-Amino-dekalin-Derivate und deren pflanzenphysiologisch verträglichen Salze als Fungizide sind sowohl die Diastereomeren bzw. die Enantiomeren als auch deren bei der Synthese anfallenden Stereoisomerengemische geeignet. Alle diese Verbindungen werden von der vorliegenden Erfindung umfaßt.

In bestimmten Fällen läßt sich das 2-Amino-trans-dekalin-Derivat der Formel II gezielt herstellen oder isolieren.

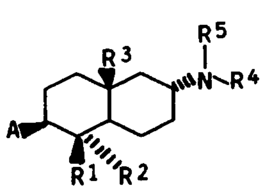

II

Verbindungen aus dieser Diastereomeren-Reihe werden besonders bevorzugt.

Die neuen Verbindungen können Verwendung als Fungizide finden.

Die Erfindung umfaßt auch Verfahren zur Herstellung der neuen N-alkylierten 2-Amino-dekaline.

Man kann die 2-Amino-dekalin-Derivate z.B. aus primären Aminen der Formel III z. B. durch schrittweise Alkylierung in an sich bekannter Weise erhalten.

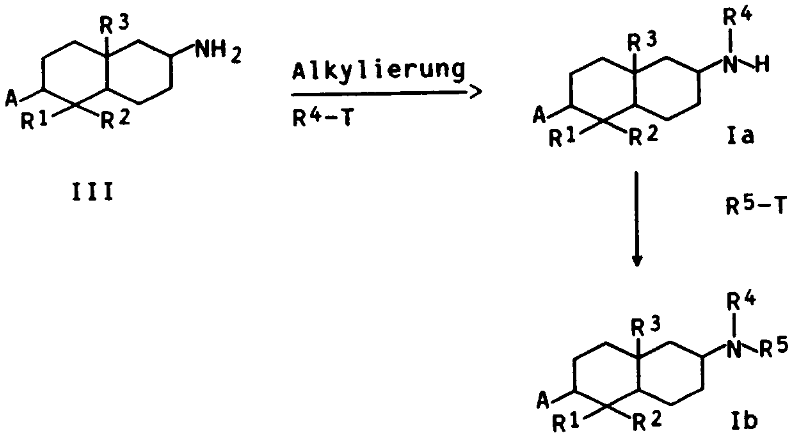


III

Ia

Ib

Als Alkylierungsmittel sind z. B. Verbindungen des Typs $R^4$-T bzw. $R^5$-T geeignet, wobei T für eine elektronenanziehende Austrittsgruppe steht. Man kann anstelle der Verbindungen des vorstehenden Typs in manchen Fällen auch Aldehyde oder Ketone verwenden, wobei diese dann den allgemeinen Formeln

$R^{13}$-CHO

und

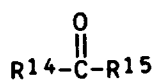

entsprechen, in welchem $R^{13}$, $R^{14}$, $R^{15}$ den Resten $R^4$ bzw. $R^5$ mit der Maßgabe entsprechen, daß sie ein C-Atom weniger als $R^4$, $R^5$ aufweisen.

Die Aldehyde bzw. Ketone werden z.B. in Gegenwart eines Reduktionsmittels und gegebenenfalls in Gegenwart eines Katalysators sowie ggf. in Gegenwart eines Lösungsmittels mit den entsprechenden primären bzw. sekundären Aminen umgesetzt.

Eine andere Möglichkeit zur Herstellung der erfindungsgemäßen Verbindungen besteht beispielsweise darin, daß man ein $\beta$-Dekalon-Derivat der Formel IV entweder einstufig oder über die Imin-/Iminiumsalzstufe V unter Verwendung eines Reduktionsmittels, ggf. in Gegenwart eines Verdünnungsmittels mit einem entsprechend substituierten Amin der Formel VI aminiert,

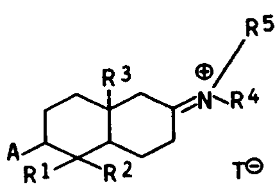

V

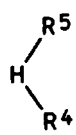

VI

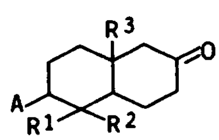

IV

wobei die Reste A und $R^1$ - $R^5$ dieselbe Bedeutung wie in I haben und $T^{\ominus}$ ein beliebiges Anion darstellt.

Die als Ausgangsstoffe benötigten Verbindungen der Formel $R^4$-T bzw. $R^5$-T in denen T für Chlor, Brom und Iod, oder für jeweils gegebenenfalls substituiertes Alkyl- oder Arylsulfonyloxy, insbesondere für Methansulfonyloxy, Trifluormethansulfonyloxy oder p-Toluolsulfonyloxy steht, sind bekannte und vielfach handelsübliche Verbindungen oder sie sind auf bekanntem Weg aus den entsprechenden Alkoholen herstellbar.

Als Verdünnungsmittel zur Durchführung der Alkylierung mit Verbindungen des Typs $R^4$-T, $R^5$-T kommen sowohl protonische als auch aprotische Lösungsmittel in Frage.

Hierzu gehören insbesondere Alkohole wie Methanol, Ethanol, Propanol, Butanol, Amylalkohol, aliphatische oder aromatische, gegebenenfalls halogenierte Kohlenwasserstoffe, wie beispielsweise Benzin, Benzol,

Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Ketone wie Aceton oder Butanon, Nitrile wie Acetonitril oder Propionitril, Amide wie Dimethylformamid, Dimethylacetamid, N-Methylformamilid, N-Methylpyrrolidon oder Hexamethylphosphorsäuretriamid, Ester wie Essigsäureethylester oder Sulfoxide, wie Dimethylsulfoxid. Es kann jedoch auch ohne Verdünnungsmittel gearbeitet werden.

Das erfindungsgemäße Verfahren verläuft in Gegenwart eines Säurebindemittels, also einer anorganischen oder organischen Base. Hierzu gehören beispielsweise Alkalimetallhydroxide, wie Natriumhydroxid oder Kaliumhydroxid; Alkalimetallcarbonate, wie Natriumcarbonat, sowie tertiäre Amine, wie Triemethylamin, N,N-Dimethylanilin, Pyridin, N,N-Dimethylaminopyridin,Diazabicycloundecan (DBU).

Ein entsprechender überschuß des reagierenden Amins kann ebenfalls als Säurebindemittel und falls das Amin flüssig ist, auch als Verdünnungsmittel dienen.

Die Umsetzungsbedingungen für solche Alkylierungen sind die einschlägig üblichen und z. B. in Methoden d. org. Chemie (Houben-Weyl), Bd. 11/1. ausführlich beschrieben.

Das gleiche gilt auch, wenn zur Durchführung des erfindungsgemäßen Verfahrens als Ausgangsstoffe Aldehyde oder Ketone verwendet werden; auch diese sind i. a. bekannte Verbindungen und vielfach im Handel erhältlich.

Die zur Durchführung der zuletzt angegebenen Verfahrensvariante benötigten Decalylimine bzw. Iminiumsalze V lassen sich mit bekannten Methoden aus β-Decalonen und Aminen herstellen.

(Vgl.: Methoden der Org. Chemie (Houben-Weyl), Bd. VIII, S. 1945 ff. 4. Aufl. G. Thieme Verlag 1952. Methoden der Org. Chemie (Houben-Weyl), Bd. XI/2, S. 77 ff., 4. Auflage, G. Thieme Verlag Stuttgart 1958. Methoden der Org. Chemie (Houben-Weyl ), Bd. VII 2b, S. 1948 ff., 4. Auflage, G. Thieme Verlag Stuttgart 1976).

Als Reduktionsmittel bei der Durchführung des erfindungsgemäßen Verfahrens werden komplexe Hydride, vorzugsweise Natriumborhydrid oder Natriumcyanborhydrid verwendet und als Verdünnungsmittel vorzugsweise Alkohole wie Methanol oder Ethanol.

Als ganz besonders vorteilhaft hat sich die Verwendung des Gemisches Natriumcyanborhydrid/Zink(2)-chlorid erwiesen. (S. Kimet et al., J. Org. Chem. 50, 1927 (1985).)

Verbindungen V lassen sich z.B. auch bequem mit Wasserstoff in Gegenwart eines Katalysators bzw. mit Ameisensäure als Reduktionsmittel nach der Methode von Leuckart-Wallach in die gewünschte 2-Aminodekaline I/II überführen.

Die zur Herstellung der erfindungsgemäßen Verbindungen benötigten 2-Aminodekaline und β-Decalone der Formeln III und IV sind teilweise kommerziell erhältlich, teilweise literaturbekannt.

Die Herstellung weiterer Ausgangsverbindungen III und IV ist in Schema 1 skizziert. Prinzipiell sind auf diesen Wegen weitere Verbindungen herstellbar.

Schema 1

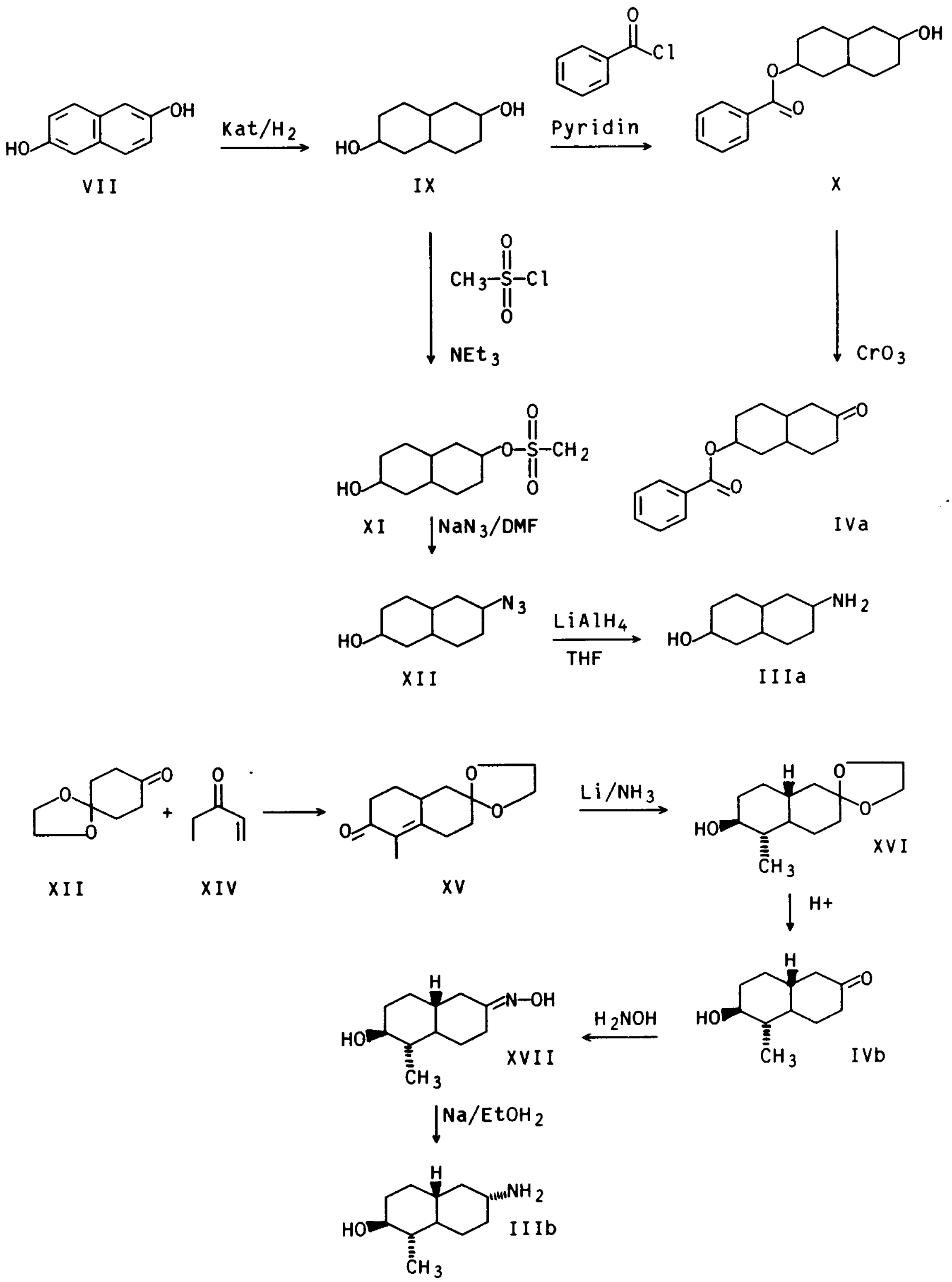


VII IX X XI IVa XII IIIa XII XIV XV XVI IVb XVII IIIb

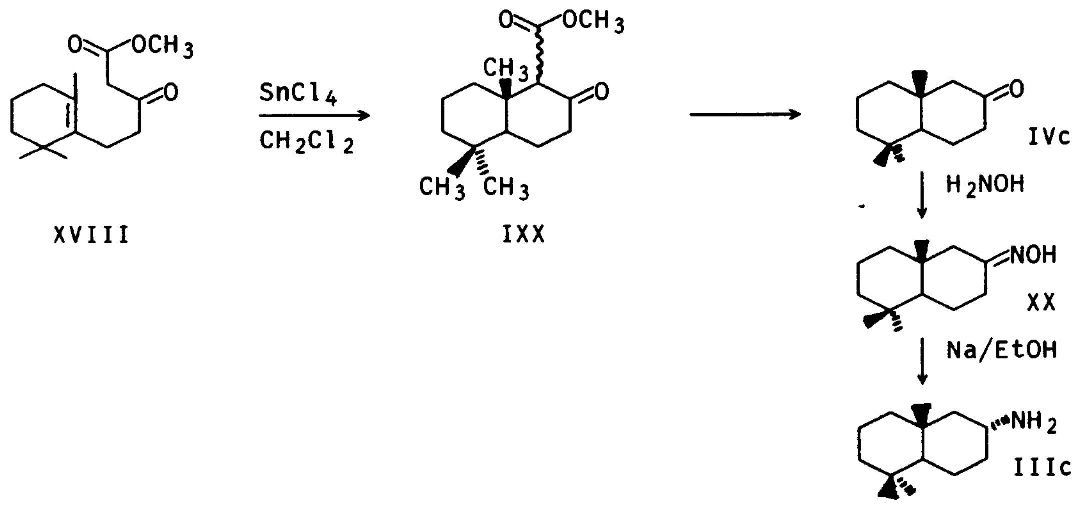


XVIII IXX IVc XX IIIc

Decalindiol IX ist bekannt (R. A. Wiley et al., J. Med. Chem. 1972, 374) und läßt sich entweder nach einer mono-Benzoylierung (mit Benzoylchlorid/Pyridin) mit Chromtrioxid auf an sich bekannte Weise zum Keton IVa oxidieren oder nach seiner Überführung in das mono-Mesylat, anschließender nucleophiler Substitution durch Natriumazid (vgl. z. B. J. A. Durden et al., J. Chem. Eng. Data 1964 (9), 228) und Reduktion, beispielsweise durch Lithiumaluminiumhydrid (vgl. z. B. J. H. Boyer et al., Chem. Rev. 1954 (54), 1), in das Amin IIIa umwandeln.

Aus Cyclohexandion-mono-ethylenketal XIII und Ethyl-vinyl-keton XIV bzw. einem geeigneten Ethylenvinylketon-Equivalent ist auf dem Wege einer Robinson-Anellierung das Octalon-dion-monoethylenketal XV zugänglich (Vgl. z. B. G. Majetich, J. Org. Chem. 1977 (42) 2327. D. Caine, J. Org. Chem. 1980 (45), 3278, S. K. Maji, J. Chem. Soc. Perk. 1, 1980, 2511; R. B. Miller, J. Am. Chem. soc. 1974, 8102).

Reduktion der $\alpha,\beta$-ungesättigten Carbonylfunktion mit Lithium in flüssigem Ammoniak, Spaltung des Ketals mit $H_2SO_4$/Kieselgel zu IVb, Überführung von IVb in das Oxim und anschließende Reduktion mit Natrium in Ethanol liefert das 6-Amino-1-methyl-2-hydroxy-trans-dekalin IIIb.

Die Verseifung und gleichzeitige Decarboxylierung von IXX (bekannt aus der Literatur: J. D. White et al., J. Org. Chem. 1985, 50, 1939) mit Kaliumhydroxid in Ethanol/Wasser liefert das bereits früher beschriebene (G. Ohloff et al., Helv. Chim. Acta 56, 1914 (1973)) Trimethyl-trans-decalon IVc, welches auf an sich bekannte Weise über das Oxim und dessen anschließende Reduktion mit Natrium in Ethanol in das Dekalinderivat IIIc mit equatorialer Aminofunktion überführt werden kann.

Die folgenden Beispiele erläutern die Herstellung der neuen Verbindungen

Herstellbeispiele

Herstellung der Ausgangsverbindungen

Vorschrift 1:

6-Benzoyloxy-decalon-2 (Verb. IVa)

Eine Lösung von 27,4 g (0,1 mol) 2-Benzoyloxy-6-hydroxy-decalin (X) in 100 ml Dichlormethan wurde unter Kühlung zu einer Mischung aus 30 g (0,3 mol) Chromtrioxid und 48 g (0,6 mol) Pyridin in 150 ml Dichlormethan getropft und 2 Std. bei Raumtemperatur (20°C)gerührt. Der überstand wurde abdekantiert, der verbliebene Rückstand mit Methyl-tert.-butylether mehrmals extrahiert. Die vereinigten org. Phasen wurden mit verd. Salzsäure, Natriumhydrogencarbonat-Lsg. und Kochsalz-Lsg. gewaschen, über Natriumsulfat getrocknet und i. Vak. eingeengt. Eine weitere Reinigung erfolgte durch Chromatographie an Kieselgel ($CH_2Cl_2$/Diethylether).
Farbloses Öl
IR (Film): 2929, 1713, 1451, 1315, 1277, 1114, 715 $cm^{-1}$

Vorschrift 2:

2-Methylsulfonyl-6-hydroxy-dekalin (Verb. XI)

Zu einer vorgelegten Mischung aus 34 g (0,2 mol) Dekalindiol und 30,3 g (0,3 mol) Triethylamin in 500 ml Dichlormethan wurden bei 0 -5°C 25,5 g (0,22 mol) Methansulfonylchlorid zugetropft. Anschließend wurde 1 Std. bei Raumtemperatur nachgerührt. Der Ansatz wurde auf Eis gegossen, die organische Phase wurde abgetrennt und die wässrige Phase mehrmals mit Dichlormethan extrahiert. Die vereinigten organischen Phasen wurden mit verd. Salzsäure, Wasser und Natriumhydrogencarbonat-Lsg. (5 %ig) gewaschen, über Natriumsulfat getrocknet und i. Vak. eingeengt.
Man erhielt 45,2 g (91 %) eines gelben, viskosen Öls.
$^1$H-NMR ($CDCl_3$):$\delta$ = 5.0-4.5(m,1H)
4.1-4.0(m,0.25H, $H_{eq}$), 3.65-3.5(m,0.75H, $H_{ax}$),
3.05-3.0(mehrere S,3H), 2.1-1.4(m,14H).

Vorschrift 3:

2-Azido-6-hydroxy-dekalin (Verbindung XII)

24,8 g (0,1 mol) Dekalindiol-mono-mesylat (XI) wurden in 1 l Dimethylformamid zusammen mit 65 g (1 mol) Natriumazid und 100 ml Wasser 5 Std. auf 90 - 100°C erhitzt. Nach dem Abkühlen wurde in 2 l Wasser gegossen, mit Methyl-tert.-butyl-ether extrahiert, die org. Extrakte wurden mit Natriumchlorid-Lsg. gewaschen, getrocknet ($Na_2SO_4$) und i. Vak. eingeengt.
17 g (88 %) eines gelblichen Öls, welches direkt weiter umgesetzt wurde.

Vorschrift 4:

2-Amino-6-hydroxy-dekalin (Verb. IIIa)

Eine Lösung von 17 g (0,09 mol) 2-Azido-6-hydroxy-dekalin (XII) in 100 ml abs. Tetrahydrofuran wurde bei 40 - 50°C zu einer Suspension von 3,8 g (0,1 mol) Lithiumaluminiumhydrid in 100 ml abs. Tetrahydrofuran getropft. Anschließend wurde 3 Std. zum Rückfluß erhitzt. Man hydrolysierte mit verd. Natronlauge (20 ml), versetzte mit Natriumsulfat und saugte ab. Das Filtrat wurde i. Vak. eingeengt. Der Rückstand wurde über das Hydrochlorid gereinigt.
Man erhielt 6 g (40 % d. Th.) von IIIa als farblosem Öl.
$^1$H-NMR ($CDCl_3$):$\delta$ = 4.1-3.4(m, 1H), 3.2-2.6(m,1H), 2.4-0.9 (m,17H).
IR (Film): 3344, 3279, 2920, 2856, 1596, 1444, 1054, 1014 $cm^{-1}$.

Vorschrift 5:

1-Methyl-6,6-ethylendioxy-Δ8a-decalon-2 (Verb. XV)

Zu einer Lösung von 22,5 g Kaliumhydroxid in 150 ml abs. Ethanol und 150 ml Diethylether wurden unter Stickstoff bei 0 - 5°C 78 g (0,5 mol) 4,4-Ethylendioxycyclohexanon (XIII) in 150 ml Diethylether zugetropft. Nach 45 Min. wurden 42 g (0,5 mol) Ethylvinylketon in 250 ml Diethylether bei 0 - 5°C innerhalb von 60 Min. zugegeben. Es wurde 3 Std. bei 20°C nachgerührt, auf Eis gegossen, die org. Phase wurde abgetrennt, die wäßrige Phase mit Methyl-tert.-butylether extrahiert. Die vereinigten organischen Phasen wurden mit Wasser gewaschen, getrocknet, i. Vak. eingeengt und destilliert: Sdp. 121 - 124°C/0,3 mbar.
Ausbeute: 23 g (26 %) eines gelben Öls.
$^1$H-NMR ($CDCl_3$):$\delta$ = 4.0(S,4H), 2.9-1.0(m,11H), 1.8(S,3H).
IR (Film): 2948, 2884, 1666, 1369, 1135, 1065 $cm^{-1}$

Vorschrift 6:

1-Methyl-2-hydroxy-6,6-ethylendioxy-dekalin (Verb. XVI)

26 g (0,12 mol) des Produkts aus Beispiel 5 wurden in 2 l flüssigem Ammoniak, 250 ml Tetrahydrofuran und 26 g (0,35 mol) tert.-Butanol vorgelegt. Innerhalb von 2 Std. wurden 19,7 g (2,1 mol) Lithium-Draht

zugefügt. Nach weiteren 15 Min. wurden 250 ml Ethanol innerhalb von 2 Std. zugetropft; es wurde vorsichtig mit 200 g festem Ammoniumchlorid versetzt und man ließ den Ammoniak über Nacht abdampfen. Der Rückstand wurde in Methyl-tert-butylether/Wasser gelöst und mit Ammoniumchlorid gesättigt. Die org. Phase wurde abgetrennt, die wäßrige Phase mit Methyl-tert.-butylether extrahiert. Übliche Aufarbeitung der organischen Extrakte ergab 23,4 g (86 %) eines hellbraunen Festkörpers.
Schmp.: 56 - 68 ° C
$^1$H-NMR ($CDCl_3$):$\delta$ = 3.92(s,4H), 3.2-3.05(m,1H) 1.05(s,3H), 2.1-0.6(m, 13H).
IR (Film): 3388, 2967, 2931, 2866, 1366, 1141, 1096, 1039 $cm^{-1}$.

Vorschrift 7:

6-Hydroxy-5-methyl-2-decalon (Verb. IVb)

19,4 g (86 mmol) des Acetals XVI aus Beispiel 6 wurden 7 Std. in 150 ml Dichlormethan mit 40 ml 15 %iger Schwefelsäure und 20 g Kieselgel bei 20 ° C gerührt. Es wurde vom Kieselgel abgesaugt und sehr gut mit Dichlormethan nachgewaschen. Die org. Phase des Filtrats wurde abgetrennt, mit 5 %iger Natriumhydrogencarbonat-Lösung gewaschen, getrocknet (Natriumsulfat) und i. Vak. eingeengt. Man erhielt 19,6 g (93 %) eines gelben Öls.
$^1$H-NMR ($CDCl_3$):$\delta$ = 3.25-3.15(m,1H), 2.45-1.0(m,13H), 1.1(d,3H)

Vorschrift 8:

trans-5,5,8a-Trimethyl-dekalon-2 (Verb. IVC)

Ein Gemisch aus 100 g (0,4 mol) 1-Carboxymethyl-5,5,8a-trimethyl-dekalon-2 (Verb. IXX), 64 g (1,6 mol) Natriumhydroxid, 900 ml Wasser und 900 ml Ethanol wurde 6 Std. zum Rückfluß erhitzt. Nach dem Abkühlen wurde mit verd. Salzsäure angesäuert, mit Dichlormethan extrahiert und wie üblich weiter aufgearbeitet. Man erhielt 76 g (98 %) eines farblosen Öls (lt. GC 99 % Produkt).
$^1$H-NMR ($CDCl_3$):$\delta$ = 2.5-1.2(m,13H), 1.0(s,3H), 0.94(s,3H), 0.88(s.3H).
IR (Film): 2957, 2921, 2944, 2843, 1712, 1963 $cm^{-1}$

Vorschrift 9:

trans-5,5,8a-Trimethyl-decalon-oxim (Verb. XX)

40 g (0,2 mol) Keton IVc aus Beispiel 8, 20,8 mol Hydroxylamin-Hydrochlorid und 16,4 g (0,2 mol) Natriumacetat wurden in 100 ml Wasser und 280 ml Ethanol 5 Std. zum Rückfluß erhitzt. Es wurde auf Eis gegossen, die org. Phase wurde abgetrennt und die wäßrige Phase gut mit Methyl-tert.-butylether extrahiert. Die weitere Aufarbeitung der organischen Phasen ergab 34 g (81 %) eines Festkörpers mit einem Schmelzpunkt von 120 - 128 ° C.
IR (KBr): 3296, 2960, 2931, 2867, 1457, 1444, 921, 912 $cm^{-1}$
Auf analoge Weise läßt sich auch das trans-6-Hydroxy-5-methyl-2-decalon-oxim XVII herstellen: 54 % Ausbeute, Schmp. 180 ° C,
$^1$H-NMR ([$d_6$]-DMSO):$\delta$ = 10.1(s,1H), 4.5(br.s,1H), 3.5-0.9(m,13H), 0.9(d,3H).

Vorschrift 10:

trans-2-Amino-5,5,8a-trimethyl-decalin (Verb. IIIc)

zu einer Lösung von 20 g (95,7 mmol) des Oxims XX aus Beispiel 9 in 360 ml Ethanol wurden unter Stickstoff portionsweise bei Rückflußtemperatur 60 g (2,6 mol) Natrium zugegeben. Es wurde so lange erhitzt, bis alles Natrium in Lösung gegangen war. Nach dem Abkühlen wurde vorsichtig mit Wasser hydrolysiert und mit Methyl-tert-butylether extrahiert. Die organischen Extrakte lieferten nach der üblichen Aufarbeitung 30 g (80 %) eines farblosen Öls, bei dem es sich lt. GC und $^1$H-NMR um 91 % des Isomeren mit equatorialer $NH_2$-Gruppe handelte.
$^1$H-NMR ($CDCl_3$):$\delta$ = 2.94-2.80(m,1H), 2.05-0.75(m,13H), 1.00(s,3H), 0.85(s,3H), 0.79(s,3H).
IR (Film): 2925, 1583, 1460, 1388, 1980, 1342 $cm^{-1}$
Auf analoge Weise läßt sich auch trans-2-Amino-6-hydroxy-5-methyl-dekalin (IIIb) herstellen (52 % Ausbeu-

te; Schmp.: 125 °C).
$^1$H-NMR ($CDCl_3$):$\delta$ = 3.2-3.0(m,1H), 2.7-2.5(m,1H), 2.1-0.5(m,16H).

Herstellung der Endprodukte

Beispiel 1:

N-3,7-Dimethyl-octyl-2-amino-dekalin (Verb. Nr. 17) (nicht erfindungsgemäß)

Eine Mischung aus 5 g (35 mmol) 2-Amino-dekalin, 7,7 g 3,7-Dimethyloctylbromid (35 mmol) und 4,8 g (35 mmol) Kaliumcarbonat in 100 ml Acetonitril wurde 8 Std. zum Rückfluß erhitzt. Das Lösungsmittel wurde i. Vak. abdestilliert, der Rückstand wurde in Dichlormethan/verd. Natronlauge aufgenommen, die organische Phase wurde abgetrennt, wie üblich aufgearbeitet und an Kieselgel aus Hexan/Methyl-tert.-butylether chromatographiert. (Physikalische Daten siehe Tabelle).

Beispiel 2

N(4-tert.-Butoxy-cyclohexyl-methyl)-5,5,8a-trimethyl-2-amino-trans-dekalin (Verb. Nr. 285) (nicht erfindungsgemäß)

2 g (10,3 mmol) trans-5,5,8a-Trimethyl-2-amino-dekalin (IIIc), 1,84 g (10,3 mmol) 4-tert.-Butoxy-1-formylcyclohexan (Isomerengemisch) und 5 g Molekularsieb (4 A) wurden über Nacht in 100 ml absolutem Methanol bei 20 °C gerührt. Dann wurden 0,8 g (20,6 mmol) Natriumborhydrid zugegeben und weitere 3 Std. bei 40 °C gerührt.

Der Ansatz wurde i. Vak. eingeengt, der Rückstand mit Wasser hydrolysiert und mit Methyl-tert.-butylether extrahiert. Die übliche Aufarbeitung ergab 1,65 g (44 %) eines farblosen Öls. Physikalische Daten vgl. Tabelle 2.

Beispiel 3

N(4-tert.-Butoxy-benzyl)N-methyl-5,5,8a-trimethyl-2-amino-trans-dekalin (Verb. Nr. 244) (nicht erfindungsgemäß)

Eine Mischung aus 2,5 g (7 mmol) N(4-tert.-Butoxy-benzyl)-5,5,8a-trimethyl-2-amino-trans-dekalin, (Verb. Nr. 243 aus Tabelle 2), 5,7 g 35 %ige Formaldehyd-Lsg. und 2,6 g (70 mmol) Natriumborhydrid in 100 ml Methanol wurde 3 Std. bei 40 °C gerührt. Der Ansatz wurde i. Vak. eingeengt, mit Wasser hydrolysiert und mit Methyl-tert.-buthyl-ether extrahiert. Die übliche Aufarbeitung des organische Extrakts lieferten 1,4 g (56 %) eines gelben Öls (lt. GC 58 %). Physikalische Daten siehe Tabelle 2.

Beispiel 4:

2(4-tert.-Butyl-piperidyl)dekalin (Verb. Nr. 129)

13,8 (0,1 mol) 4-tert.-Butylpiperidin, 7,5 g (0,05 mol) $\beta$-Dekalin, 6,9 (0,05 mol) Zink(II)chlorid und 6,8 g (0,11 mol) Natriumcyanborhydrid wurden in 100 ml Methanol 48 Std. bei 20 °C gerührt.

Der Ansatz wurde i. Vak. eingeengt, mit verd. NaOH hydrolysiert, mit Methyl-tert.-butylether extrahiert und wie üblich aufgearbeitet.

Destillation i. Vak. (130 °C/0,2 mbar) lieferten 2,4 g (17,6 %) eines farblosen Öls.

Auf die in den Beispielen 1 bis 4 beschriebene Art und Weise sind die in Tabelle 1 und 2 aufgeführten Verbindungen zugänglich.

Tabelle 1

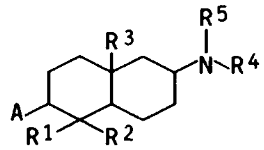

I

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 1 | H | H | H | H | H | n-Hexyl | |
| 2 | H | H | H | H | H | n-Heptyl | |
| 3 | H | H | H | H | H | n-Octyl | |
| 4 | H | H | H | H | H | n-Decyl | |
| 5 | H | H | H | H | H | n-Dodecyl | |
| 6 | H | H | H | H | $CH_3$ | n-Hexyl | |
| 7 | H | H | H | H | $CH_3$ | n-Heptyl | |
| 8 | H | H | H | H | $CH_3$ | n-Octyl | |
| 9 | H | H | H | H | $CH_3$ | n-Decyl | |
| 10 | H | H | H | H | $CH_3$ | n-Dodecyl | |
| 11 | H | H | H | H | H | 3-Methyl-5,5-dimethyl-hexyl | |
| 12 | H | H | H | H | $CH_3$ | 3-Methyl-5,5-dimethyl-hexyl | |
| 13 | H | H | H | H | $C_2H_5$ | 3-Methyl-5,5-dimethyl-hexyl | |
| 14 | H | H | H | H | H | 3-Methyl-7-methyl-hept-5-enyl | 148/0,3 mbar |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR($cm^{-1}$) |
|---|---|---|---|---|---|---|---|
| 15 | H | H | H | H | $CH_3$ | 3-Methyl-7-methyl-hept-5-enyl | |
| 16 | H | H | H | H | $C_2H_5$ | 3-Methyl-7-methyl-hept-5-enyl | |
| 17 | H | H | H | H | H | 3-Methyl-7-methyl-heptyl | 145/0,2 mbar |
| 18 | H | H | H | H | $CH_3$ | 3-Methyl-7-methyl-hept-5-enyl | |
| 19 | H | H | H | H | $C_2H_5$ | 3-Methyl-7-methyl-hept-5-enyl | |
| 20 | H | H | H | H | H | 1,5,9-Trimethyl-nonyl | |
| 21 | H | H | H | H | $CH_3$ | 1,5,9-Trimethyl-nonyl | |
| 22 | H | H | H | H | $C_2H_5$ | 1,5,9-Trimethyl-nonyl | |
| 23 | H | H | H | H | H | 3,5,5-Trimethyl-hexyl | 140/0,2 mbar |
| 24 | H | H | H | H | $CH_3$ | 3,5,5-Trimethyl-hexyl | |
| 25 | H | H | H | H | $C_2H_5$ | 3,5,5-Trimethyl-hexyl | |
| 26 | H | H | H | H | H | 2,3,5,5-Tetramethyl-hexyl | |
| 27 | H | H | H | H | $CH_3$ | 2,3,5,5-Tetramethyl-hexyl | |
| 28 | H | H | H | H | H | 2,4,5,7,7-Pentamethyl-octyl | |
| 29 | H | H | H | H | $CH_3$ | 2,4,5,7,7-Pentamethyl-octyl | |
| 30 | H | H | H | H | H | 1,4-Dimethyl-heptyl | 130/0,2 mbar |
| 31 | H | H | H | H | $CH_3$ | 1,4-Dimethyl-heptyl | |
| 32 | H | H | H | H | H | Tridecyl (Isomerengemisch) | |
| 33 | H | H | H | H | $CH_3$ | Tridecyl (Isomerengemsich) | |
| 34 | H | H | H | H | $C_2H_5$ | Tridecyl (Isomerengemisch) | |
| 35 | H | H | H | H | H | p-tert.Butyl-phenyl | |
| 36 | H | H | H | H | $CH_3$ | p-tert.Butyl-phenyl | |
| 37 | H | H | H | H | $C_2H_5$ | p-tert.Butyl-phenyl | |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 38 | H | H | H | H | H | 4,4-Dimethyl-5-oxa-hexyl | |
| 39 | H | H | H | H | $CH_3$ | 4,4-Dimethyl-5-oxa-hexyl | |
| 40 | H | H | H | H | H | 4,4-Dimethyl-5-oxa-hexyl | |
| 41 | H | H | H | H | H | 4,4-Dimethyl-5-oxa-hex-2-inyl | |
| 42 | H | H | H | H | $CH_3$ | 4,4-Dimethyl-5-oxa-hex-2-inyl | |
| 43 | H | H | H | H | H | 2,4,4-Trimethyl-3-oxa-pentyl | |
| 44 | H | H | H | H | $CH_3$ | 2,5,5-Trimethyl-3-oxa-hexyl | |
| 44a | H | H | H | H | H | 2,5,5-Trimethyl-3-oxa-hexyl | |
| 45 | H | H | H | H | $CH_3$ | 2,5,5-Trimethyl-3-oxa-hexyl | |
| 46 | H | H | H | H | H | 2,3,5,5-Tetramethyl-4-oxa-hexyl | |
| 47 | H | H | H | H | $CH_3$ | 2,3,5,5-Tetramethyl-4-oxa-hexyl | |
| 48 | H | H | H | H | H | 5,5-Dimethyl-4-oxa-hexyl | |
| 49 | H | H | H | H | $CH_3$ | 5,5-Dimethyl-4-oxa-hexyl | |
| 50 | H | H | H | H | H | 6,6-Dimethyl-5-oxa-heptyl | |
| 51 | H | H | H | H | $CH_3$ | 6,6-Dimethyl-5-oxa-heptyl | |
| 52 | H | H | H | H | H | p-tert.Butyl-benzyl | 160/0,15 |
| 53 | H | H | H | H | $CH_3$ | p-tert.Butyl-benzyl | 2926, 2854, 2785, 1513, 1464, 1448, 1363, 1268, 1111, 546 |
| 54 | H | H | H | H | $C_2H_5$ | p-tert.Butyl-benzyl | |
| 55 | H | H | H | H | n-$C_3H_7$ | p-tert.Butyl-benzyl | |
| 56 | H | H | H | H | iso-$C_3H_7$ | p-tert.Butyl-benzyl | |
| 57 | H | H | H | H | Cyclopropyl | p-tert.Butyl-benzyl | 155/0,2 |
| 58 | H | H | H | H | H | p-tert.Butyloxy-benzyl | 2976, 2922, 2857, 1505, 1460, 1447, 1365, 1234, 1161, 898 |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 59 | H | H | H | H | $CH_3$ | p-tert.Butyloxy-benzyl | |
| 60 | H | H | H | H | $C_2H_5$ | p-tert.Butyloxy-benzyl | |
| 61 | H | H | H | H | n-$C_3H_7$ | p-tert.Butyloxy-benzyl | |
| 62 | H | H | H | H | iso-$C_3H_7$ | p-tert.Butyloxy-benzyl | |
| 63 | H | H | H | H | Cyclopropyl | p-tert.Butyloxy-benzyl | |
| 64 | H | H | H | H | H | p-1,1-Dimethylpropyl-benzyl | |
| 65 | H | H | H | H | $CH_3$ | p-1,1-Dimethylpropyl-benzyl | |
| 66 | H | H | H | H | $C_2H_5$ | p-1,1-Dimethylpropyl-benzyl | |
| 67 | H | H | H | H | H | p-1,1,2-Trimethylpropyl-benzyl | |
| 68 | H | H | H | H | $CH_3$ | p-1,1,2-Trimethylpropyl-benzyl | |
| 69 | H | H | H | H | H | p-1,1,3,3-Tetramethylbutyl-benzyl | 2921,2856,1512,1465,1448,1394,1365, 1108,1018,819 |
| 70 | H | H | H | H | $CH_3$ | p-1,1,3,3-Tetramethylbutyl-benzyl | |
| 71 | H | H | H | H | H | 3-(p-tert.Butyl-phenyl)-ethyl | 2924,2858,1512,1463,1447,1363,1121, 1109,833,821 |
| 72 | H | H | H | H | $CH_3$ | 3-(p-tert.Butyl-phenyl)-ethyl | |
| 73 | H | H | H | H | $C_2H_5$ | 3-(p-tert.Butyl-phenyl)-ethyl | |
| 74 | H | H | H | H | n-$C_3H_7$ | 3-(p-tert.Butyl-phenyl)-ethyl | 2955,2923,2860,2807,1515,1463, 1447,1363,1268,826 |
| 75 | H | H | H | H | H | 3-Phenyl-propyl | 176-180/1,0 mbar |
| 76 | H | H | H | H | $CH_3$ | 3-Phenyl-propyl | 176-178/0,5 mbar |
| 77 | H | H | H | H | $C_2H_5$ | 3-Phenyl-propyl | 180/2,0 mbar |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 78 | H | H | H | H | n-$C_3H_7$ | 3-Phenyl-propyl | 188-190/2,0 mbar |
| 79 | H | H | H | H | i-propyl | 3-Phenyl-propyl | 180-183/1,0 mbar |
| 80 | H | H | H | H | H | 3-(p-tert.Butyl-phenyl)-propyl | 246-250/3,0 mbar |
| 81 | H | H | H | H | $CH_3$ | 3-(p-tert.Butyl-phenyl)-propyl | 200-204/0,8 mbar |
| 82 | H | H | H | H | $C_2H_5$ | 3-(p-tert.Butyl-phenyl)-propyl | |
| 83 | H | H | H | H | i-propyl | 3-(p-tert.Butyl-phenyl)-propyl | 210-216/1,0 mbar |
| 84 | H | H | H | H | H | 2-Methyl-3-(p-tert.butyl-phenyl)-propyl | 200-220/0,5 mbar |
| 85 | H | H | H | H | $CH_3$ | 2-Methyl-3-(p-tert.butyl-phenyl)-propyl | |
| 86 | H | H | H | H | H | 2-Methyl-3-(p-tert.butyl-o-methyl-phenyl)-propyl | |
| 87 | H | H | H | H | $CH_3$ | 2-Methyl-3-(p-tert.butyl-o-methyl-phenyl)-propyl | |
| 88 | H | H | H | H | H | p-tert.Butylthio-benzyl | |
| 89 | H | H | H | H | $CH_3$ | p-tert.Butylthio-benzyl | |
| 90 | H | H | H | H | H | p-Trimethylsilyl-benzyl | |
| 91 | H | H | H | H | $CH_3$ | p-Trimethylsilyl-benzyl | |
| 92 | H | H | H | H | H | p-(1-methoxy-1-methyl-ethyl)-benzyl | |
| 93 | H | H | H | H | $CH_3$ | p-(1-methoxy-1-methyl-ethyl)-benzyl | |

Tabelle 1

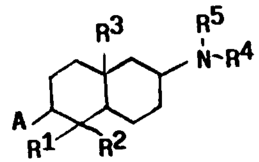

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| | | | | | | hexyl)-propyl | |
| 108 | H | H | H | H | H | 3-tert.Butyl-isoxazol-5-methyl | 2922, 2857, 2963, 1464, 1448, 1366, 1606, 800, 1410, 1218 $cm^{-1}$ |
| 109 | H | H | H | H | $CH_3$ | 3-tert.Butyl-isoxazol-5-methyl | 2923, 2858, 2962, 1464, 1448, 1365, 1604, 2791, 1208, 1408 $cm^{-1}$ |

Tabelle 1 (Fortsetzung)

| Verb. Nr. | A | $R^1$ | $R^2$ | $R^3$ | $R^4$ | R5 | Physikalische Daten: Schmp., Sdp. (°C); IR ($cm^{-1}$) |
|---|---|---|---|---|---|---|---|
| 110 | H | H | H | H | H | 3-Heptyl-isoxazol-5-methyl | |
| 111 | H | H | H | H | $CH_3$ | 3-Heptyl-isoxazol-5-methyl | |
| 112 | H | H | H | H | H | 1-Tert.Butyl-pyrrol-3-methyl | |
| 113 | H | H | H | H | $CH_3$ | 1-Tert.Butyl-pyrrol-3-methyl | |
| 116 | H | H | H | H | H | 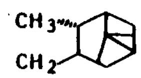 | 2923, 2858, 1448, 1466, 1373, 1384, 1123, 1148, 733 $cm^{-1}$ |
| 117 | H | H | H | H | $CH_3$ | 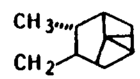 | 2923, 2860, 1448, 1465, 2784, 1367, 1383 1030, 1045, 1146 $cm^{-1}$ |
| 118 | H | H | H | H | H | 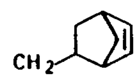 | 2923, 2859, 1447, 719, 707, 1464, 1336, 1117, 1149 $cm^{-1}$ |
| 119 | H | H | H | H | $CH_3$ | 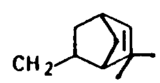 | 2923, 2860, 1447, 2779, 718, 706, 1464, 1336, 1027, 1046 $cm^{-1}$ |
| 120 | H | H | H | H | H | 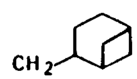 | 2922, 2860, 1463, 2447, 1365, 1381, 1117, 1148, 1060, 735 $cm^{-1}$ |
| 121 | H | H | H | H | $CH_3$ | 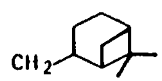 | 2924, 2862, 1448, 1461, 2780, 1365, 1048, 1381, 1024, 1122 $cm^{-1}$ |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 122 | H | H | H | H | H | $CH_2-CH_2$ 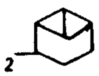 | 2919, 2858, 2980, 1465, 1447, 1366, 1382, 1148, 1117, 734 $cm^{-1}$ |
| 123 | H | H | H | H | | 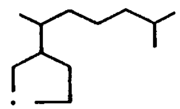 | 2924, 2860, 2978, 1465, 1947, 2787, 1366, 1382, 1026, 1047 $cm^{-1}$ |
| 124 | H | H | H | H | | 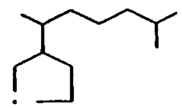 | 3289, 2923, 2855, 2783, 1466, 1447, 1377, 1366, 1150, 1058 $cm^{-1}$ |
| 125 | H | H | H | H | | 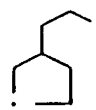 | 2923, 2863, 2777, 1718, 1476, 1465, 1376, 1364, 1161 $cm^{-1}$ |
| 126 | H | H | H | H | | 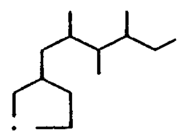 | 2924, 2863, 2777, 1718, 1476, 1465, 1392, 1376, 1152 $cm^{-1}$ |

Tabelle 1 (Fortsetzung)

| Verb. Nr. | A | $R^1$ | $R^2$ | $R^3$ | $R^4$ $R^5$ | Physikalische Daten: Schmp., Sdp. (°C); IR ($cm^{-1}$) |
|---|---|---|---|---|---|---|
| 129 | H | H | H | H | 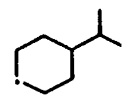 | 130/0,2 mbar |
| 130 | H | H | H | H | 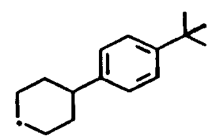 | 2973, 2851, 2795, 2742, 1463, 1446, 1378, 1362, 830, 571 $cm^{-1}$ |
| 132 | H | H | H | H | 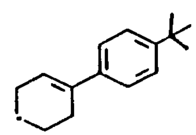 | 2920, 2854, 2793, 1460, 1447, 1379, 1361, 1270, 841, 817 |
| 133 | H | H | H | H | 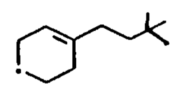 | 2921, 2859, 2795, 1465, 1447, 1391, 1364, 1244, 1150, 1114 |

Tabelle 1 (Fortsetzung)

| Verb. Nr. | A | $R^1$ | $R^2$ | $R^3$ | $R^4$ $R^5$ | Physikalische Daten: Schmp., Sdp. (°C); IR ($cm^{-1}$) |
|---|---|---|---|---|---|---|
| 134 | H | H | H | H | 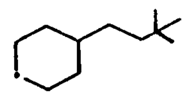 | |
| 137 | H | H | H | H | 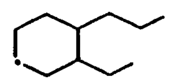 | 198/0,2mbar |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 141 | H | H | H | H | | 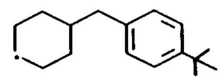 | 2921, 2857, 2796, 2744, 1464, 1447, 1363, 1269, 1109, 1067180-200/1,0 mbar |
| 142 | H | H | H | H | | 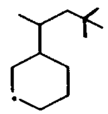 | 180-200/0,1 mbar |
| 143 | H | H | H | H | | 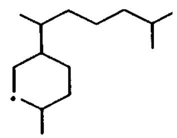 | 2951, 2921, 2858, 1465, 1448, 1375, 1366, 1240, 1212, 1166 |
| 144 | OH | H | H | H | H | p-tert.Butyl-benzyl | 2925, 2861, 1464, 1445, 1362, 1268, 1109, 1052, 831, 817 |
| 145 | OH | H | H | H | $CH_3$ | p-tert.Butyl-benzyl | |
| 146 | OH | H | H | H | H | p-tert.Butyloxy-benzyl | |
| 147 | OH | H | H | H | $CH_3$ | p-tert.Butyloxy-benzyl | |
| 148 | OH | H | H | H | H | p-(1-Methoxy-1-methyl-ethyl)-benzyl | |
| 149 | OH | H | H | H | $CH_3$ | p-(1-Methoxy-1-methyl-ethyl)-benzyl | |
| 150 | OH | H | H | H | H | p-tert.Butyl-cyclohexyl-methyl | |
| 151 | OH | H | H | H | $CH_3$ | p-tert.Butyl-cyclohexyl-methyl | |
| 152 | OH | H | H | H | H | p-tert.Butyloxy-cyclohexyl-methyl | |
| 153 | OH | H | H | H | $CH_3$ | p-tert.Butyloxy-cyclohexyl-methyl | |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 154 | OH | H | H | H | H | p-tert.Butyl-cyclohexyl-methyl | |
| 158 | $C_6H_5\overset{O}{\overset{\|}{C}}-O$ | H | H | H | H | p-tert.Butyl-benzyl | 2949, 2925, 2865, 1715, 1450, 1314, 1275, 1112, 1026, 712 |
| 159 | $C_6H_5\overset{O}{\overset{\|}{C}}-O$ | H | H | H | $CH_3$ | p-tert.Butyl-benzyl | |

Tabelle 1 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 160 | $C_6H_5\overset{O}{\overset{\|}{C}}-O$ | H | H | H | H | p-tert.Butyloxy-benzyl | 3017, 2962, 2922, 2860, 1513, 1461, 1452, 1362, 1113, 818 |
| 161 | $C_6H_5\overset{O}{\overset{\|}{C}}-O$ | H | H | H | $CH_3$ | p-tert.Butyloxy-benzyl | |
| 162 | $C_6H_5\overset{O}{\overset{\|}{C}}-O$ | H | H | H | | 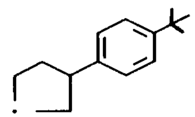 | 2932, 2867, 1716, 1450, 1314, 1275, 1112, 1069, 1026, 712 |
| 163 | $C_6H_5\overset{O}{\overset{\|}{C}}-O$ | H | H | H | | 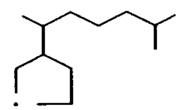 | 2950, 2928, 2868, 1717, 1451, 1315, 1277, 1113, 1070, 712 |
| 164 | $C_6H_5\overset{O}{\overset{\|}{C}}-O$ | H | H | H | | 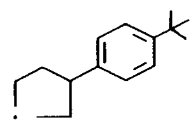 | |
| 165 | H | $CH_3$ | $CH_3$ | $CH_3$ | | 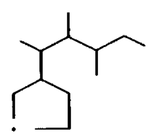 | 2949, 2866, 2844, 2775, 1715, 1461, 1388, 1382, 1216 |

Tabelle 1 (Fortsetzung)

| Verb. Nr. | A | $R^1$ | $R^2$ | $R^3$ | $R^4$ $R^5$ | Physikalische Daten: Schmp., Sdp. (°C); IR ($cm^{-1}$) |
|---|---|---|---|---|---|---|
| 166 | H | $CH_3$ | $CH_3$ | $CH_3$ | 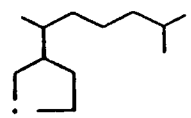 | 3317, 2952, 2927, 2867, 2786, 1462, 1381, 1366, 1131, 1058 |
| 167 | H | $CH_3$ | $CH_3$ | $CH_3$ | 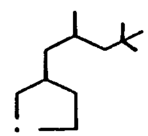 | |
| 170 | H | $CH_3$ | $CH_3$ | $CH_3$ | 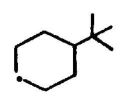 | |
| 171 | H | $CH_3$ | $CH_3$ | $CH_3$ | 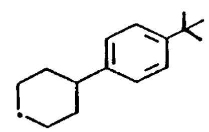 | |

Tabelle 1 (Fortsetzung)

| Verb. Nr. | A | $R^1$ | $R^2$ | $R^3$ | $R^4$ $R^5$ | Physikalische Daten: Schmp., Sdp. (°C); IR ($cm^{-1}$) |
|---|---|---|---|---|---|---|
| 173 | H | $CH_3$ | $CH_3$ | $CH_3$ | 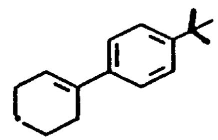 | |
| 174 | H | $CH_3$ | $CH_3$ | $CH_3$ | 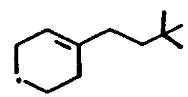 | |
| 175 | H | $CH_3$ | $CH_3$ | $CH_3$ | 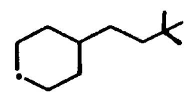 | |
| 178 | H | $CH_3$ | $CH_3$ | $CH_3$ | 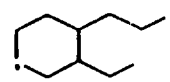 | |

Tabelle 1 (Fortsetzung)

| Verb. Nr. | A | $R^1$ | $R^2$ | $R^3$ | $R^4$ $R^5$ | Physikalische Daten: Schmp., Sdp. (°C); IR ($cm^{-1}$) |
|---|---|---|---|---|---|---|
| 182 | H | $CH_3$ | $CH_3$ | $CH_3$ | 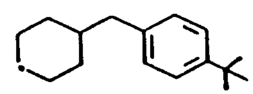 | |
| 183 | H | $CH_3$ | $CH_3$ | $CH_3$ | 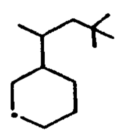 | |
| 184 | H | $CH_3$ | $CH_3$ | $CH_3$ | 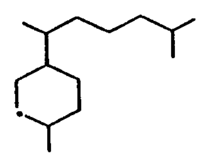 | |

Tabelle 2

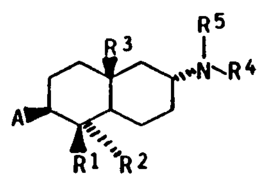

Trans-isomere

II

| Verb. Nr. | A | $R^1$ | $R^2$ | $R^3$ | $R^4$ | $R^5$ | Physikalische Daten: Schmp., Sdp. (°C); IR($cm^{-1}$) |
|---|---|---|---|---|---|---|---|

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 198 | H | $CH_3$ | $CH_3$ | $CH_3$ | H | 3-Methyl-7-methyl-hept-5-enyl | 2924, 2866, 2845, 1460, 1366, 1204, 1124, 1086, 972 |
| 199 | H | $CH_3$ | $CH_3$ | $CH_3$ | $CH_3$ | 3-Methyl-7-methyl-hept-5-enyl | |
| 200 | H | $CH_3$ | $CH_3$ | $CH_3$ | $C_2H_5$ | 3-Methyl-7-methyl-hept-5-enyl | |
| 201 | H | $CH_3$ | $CH_3$ | $CH_3$ | H | 3-Methyl-7-methyl-heptyl | Öl |
| 202 | H | $CH_3$ | $CH_3$ | $CH_3$ | $CH_3$ | 3-Methyl-7-methyl-heptyl | |
| 203 | H | $CH_3$ | $CH_3$ | $CH_3$ | $C_2H_5$ | 3-Methyl-7-methyl-heptyl | |
| 204 | H | $CH_3$ | $CH_3$ | $CH_3$ | H | 1,5,9-Trimethyl-nonyl | 3353, 2954, 2926, 2868, 2845, 1462, 1378, 1366, 1122, 1018 |
| 205 | H | $CH_3$ | $CH_3$ | $CH_3$ | $CH_3$ | 1,5,9-Trimethyl-nonyl | |
| 206 | H | $CH_3$ | $CH_3$ | $CH_3$ | $C_2H_5$ | 1,5,9-Trimethyl-nonyl | |
| 207 | H | $CH_3$ | $CH_3$ | $CH_3$ | n-$C_3H_7$ | 1,5,9-Trimethyl-nonyl | |
| 208 | H | $CH_3$ | $CH_3$ | $CH_3$ | H | 3,5,5-Trimethyl-hexyl | |
| 209 | H | $CH_3$ | $CH_3$ | $CH_3$ | $CH_3$ | 3,5,5-Trimethyl-hexyl | |
| 210 | H | $CH_3$ | $CH_3$ | $CH_3$ | $C_2H_5$ | 3,5,5-Trimethyl-hexyl | |
| 211 | H | $CH_3$ | $CH_3$ | $CH_3$ | H | 2,3,5,5-Tetramethyl-hexyl | 2952, 2931, 2867, 2843, 1462, 1388, 1379, 1125 |
| 212 | H | $CH_3$ | $CH_3$ | $CH_3$ | $CH_3$ | 2,3,5,5-Tetramethyl-hexyl | |
| 213 | H | $CH_3$ | $CH_3$ | $CH_3$ | H | 2,4,5,7,7-Pentamethyl-octyl | 2951, 2928, 2867, 2844, 1666, 1462, 1380, 1365, 1123 |
| 214 | H | $CH_3$ | $CH_3$ | $CH_3$ | $CH_3$ | 2,4,5,7,7-Pentamethyl-octyl | |
| 215 | H | $CH_3$ | $CH_3$ | $CH_3$ | H | 1,4-Dimethyl-heptyl | |
| 216 | H | $CH_3$ | $CH_3$ | $CH_3$ | $CH_3$ | 1,4-Dimethyl-heptyl | |

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 217 | H | $CH_3$ | $CH_3$ | $CH_3$ | H | Tridecyl (Isomerengemisch) | |
| 218 | H | $CH_3$ | $CH_3$ | $CH_3$ | $CH_3$ | Tridecyl (Isomerengemisch) | |
| 219 | H | $CH_3$ | $CH_3$ | $CH_3$ | $C_2H_5$ | Tridecyl (Isomerengemisch) | |
| 220 | H | $CH_3$ | $CH_3$ | $CH_3$ | H | p-tert.Butyl-cyclohexyl | |
| 221 | H | $CH_3$ | $CH_3$ | $CH_3$ | $CH_3$ | p-tert.Butyl-cyclohexyl | |
| 222 | H | $CH_3$ | $CH_3$ | $CH_3$ | $C_2H_5$ | p-tert.Butyl-cyclohexyl | |
| 223 | H | $CH_3$ | $CH_3$ | $CH_3$ | H | 4,4-Dimethyl-5-oxa-hexyl | |
| 224 | H | $CH_3$ | $CH_3$ | $CH_3$ | $CH_3$ | 4,4-Dimethyl-5-oxa-hexyl | |
| 225 | H | $CH_3$ | $CH_3$ | $CH_3$ | H | 4,4-Dimethyl-5-oxa-hex-2-inyl | |
| 226 | H | $CH_3$ | $CH_3$ | $CH_3$ | $CH_3$ | 4,4-Dimethyl-5-oxa-hex-2-inyl | |
| 227 | H | $CH_3$ | $CH_3$ | $CH_3$ | H | 2,4,4-Trimethyl-3-oxa-pentyl | |
| 228 | H | $CH_3$ | $CH_3$ | $CH_3$ | $CH_3$ | 2,4,4-Trimethyl-3-oxa-pentyl | |
| 229 | H | $CH_3$ | $CH_3$ | $CH_3$ | H | 2,5,5-Trimethyl-3-oxa-hexyl | |
| 230 | H | $CH_3$ | $CH_3$ | $CH_3$ | $CH_3$ | 2,5,5-Trimethyl-3-oxa-hexyl | |
| 231 | H | $CH_3$ | $CH_3$ | $CH_3$ | H | 2,3,5,5,-Tetramethyl-4-oxa-hexyl | |
| 232 | H | $CH_3$ | $CH_3$ | $CH_3$ | $CH_3$ | 2,3,5,5,-Tetramethyl-4-oxa-hexyl | |
| 233 | H | $CH_3$ | $CH_3$ | $CH_3$ | H | 5,5-Dimethyl-4-oxa-hexyl | |
| 234 | H | $CH_3$ | $CH_3$ | $CH_3$ | $CH_3$ | 5,5-Dimethyl-4-oxa-hexyl | |
| 235 | H | $CH_3$ | $CH_3$ | $CH_3$ | H | 6,6-Dimethyl-5-oxa-heptyl | |
| 236 | H | $CH_3$ | $CH_3$ | $CH_3$ | $CH_3$ | 6,6-Dimethyl-5-oxa-heptyl | |
| 237 | H | $CH_3$ | $CH_3$ | $CH_3$ | H | p-tert.Butyl-benzyl | 2994, 2961, 2948, 2926, 2906, 2866, 1461, 13860, 1365 |
| 238 | H | $CH_3$ | $CH_3$ | $CH_3$ | $CH_3$ | p-tert.Butyl-benzyl | |

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 239 | H | $CH_3$ | $CH_3$ | $CH_3$ | $C_2H_5$ | p-tert.Butyl-benzyl | |
| 240 | H | $CH_3$ | $CH_3$ | $CH_3$ | n-$C_3H_7$ | p-tert.Butyl-benzyl | |
| 241 | H | $CH_3$ | $CH_3$ | $CH_3$ | iso-$C_3H_4$ | p-tert.Butyl-benzyl | |
| 242 | H | $CH_3$ | $CH_3$ | $CH_3$ | Cyclopropyl | p-tert.Butyl-benzyl | |
| 243 | H | $CH_3$ | $CH_3$ | $CH_3$ | H | p-tert.Butyloxy-benzyl | 2975, 2928, 2866, 2843, 1506, 1460, 1365, 1234, 1161, 898 |
| 244 | H | $CH_3$ | $CH_3$ | $CH_3$ | $CH_3$ | p-tert.Butyloxy-benzyl | 2974, 2930, 2866, 2843, 1504, 1365, 1233, 1172, 1160, 899 $cm^{-1}$ |
| 245 | H | $CH_3$ | $CH_3$ | $CH_3$ | $C_2H_5$ | p-tert.Butyloxy-benzyl | |
| 246 | H | $CH_3$ | $CH_3$ | $CH_3$ | n-$C_3H_7$ | p-tert.Butyloxy-benzyl | |
| 247 | H | $CH_3$ | $CH_3$ | $CH_3$ | Allyl | p-tert.Butyloxy-benzyl | Öl |
| 248 | H | $CH_3$ | $CH_3$ | $CH_3$ | Propargyl | p-tert.Butyloxy-benzyl | Öl |
| 249 | H | $CH_3$ | $CH_3$ | $CH_3$ | H | p-1,1-Dimethylpropyl-benzyl | 2963, 2925, 2878, 2867, 1461, 1387, 1379, 1124 |
| 250 | H | $CH_3$ | $CH_3$ | $CH_3$ | $CH_3$ | p-1,1-Dimethylpropyl-benzyl | |
| 251 | H | $CH_3$ | $CH_3$ | $CH_3$ | $C_2H_5$ | p-1,1-Dimethylpropyl-benzyl | |
| 252 | H | $CH_3$ | $CH_3$ | $CH_3$ | H | p-1,1,2-Trimethylpropyl-benzyl | 2963, 2927, 2875, 2843, 1461, 1388, 1378, 1124, 1110 |
| 253 | H | $CH_3$ | $CH_3$ | $CH_3$ | $CH_3$ | p-1,1,2-Trimethylpropyl-benzyl | |
| 254 | H | $CH_3$ | $CH_3$ | $CH_3$ | H | p-1,1,3,3-Tetramethylbutyl-benzyl | 154-70/0,3 mbar |
| 255 | H | $CH_3$ | $CH_3$ | $CH_3$ | $CH_3$ | p-1,1,3,3-Tetramethylbutyl-benzyl | |
| 256 | H | $CH_3$ | $CH_3$ | $CH_3$ | H | 3-(p-tert.Butyl-phenyl)-ethyl | |
| 257 | H | $CH_3$ | $CH_3$ | $CH_3$ | $CH_3$ | 3-(p-tert.Butyl-phenyl)-ethyl | |

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 258 | H | $CH_3$ | $CH_3$ | $CH_3$ | $C_2H_5$ | 3-(p-tert.Butyl-phenyl)-ethyl | |
| 259 | H | $CH_3$ | $CH_3$ | $CH_3$ | $n-C_3H_7$ | 3-(p-tert.Butyl-phenyl)-ethyl | |
| 260 | H | $CH_3$ | $CH_3$ | $CH_3$ | H | 3-Phenyl-propyl | |
| 261 | H | $CH_3$ | $CH_3$ | $CH_3$ | $CH_3$ | 3-Phenyl-propyl | |
| 262 | H | $CH_3$ | $CH_3$ | $CH_3$ | $C_2H_5$ | 3-Phenyl-propyl | |
| 263 | H | $CH_3$ | $CH_3$ | $CH_3$ | $n-C_3H_7$ | 3-Phenyl-propyl | |
| 264 | H | $CH_3$ | $CH_3$ | $CH_3$ | i-propyl | 3-Phenyl-propyl | |
| 265 | H | $CH_3$ | $CH_3$ | $CH_3$ | H | 3-(tert.Butyl-phenyl)-propyl | |
| 266 | H | $CH_3$ | $CH_3$ | $CH_3$ | $CH_3$ | 3-(tert.Butyl-phenyl)-propyl | |
| 267 | H | $CH_3$ | $CH_3$ | $CH_3$ | $C_2H_5$ | 3-(tert.Butyl-phenyl)-propyl | |
| 268 | H | $CH_3$ | $CH_3$ | $CH_3$ | i-propyl | 3-(tert.Butyl-phenyl)-propyl | |
| 269 | H | $CH_3$ | $CH_3$ | $CH_3$ | H | 2-Methyl-3-(p-tert.butyl-phenyl)-propyl | 2994, 2960, 2926, 2867, 2843, 1461 1379, 1365, 1124, 1109 |
| 270 | H | $CH_3$ | $CH_3$ | $CH_3$ | $CH_3$ | 2-Methyl-3-(p-tert.butyl-phenyl)-propyl | |
| 271 | H | $CH_3$ | $CH_3$ | $CH_3$ | H | 2-Methyl-3-(p-tert.butyl-o-methyl-phenyl)-propyl | 2949, 2927, 2866, 2843, 1506, 1461, 1388, 1379, 1362, 1125 |
| 272 | H | $CH_3$ | $CH_3$ | $CH_3$ | $CH_3$ | 2-Methyl-3-(p-tert.butyl-o-methyl-phenyl)-propyl | |
| 273 | H | $CH_3$ | $CH_3$ | $CH_3$ | H | p-tert.Butylthio-benzyl | 2959/2922, 2865, 2842, 1471, 1457, 1380, 1363, 1168 |
| 274 | H | $CH_3$ | $CH_3$ | $CH_3$ | $CH_3$ | p-tert.Butylthio-benzyl | |
| 275 | H | $CH_3$ | $CH_3$ | $CH_3$ | H | p-Trimethylsilyl-benzyl | |

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 293 | H | $CH_3$ | $CH_3$ | $CH_3$ | H | 3-tert.Butyl-isoxazol-5-methyl | 2962, 2925, 2867, 2844, 1606, 1462, 1388, 1380, 1126 |

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 294 | H | $CH_3$ | $CH_3$ | $CH_3$ | $CH_3$ | 3-tert.Butyl-isoxazol-5-methyl | |
| 295 | H | $CH_3$ | $CH_3$ | $CH_3$ | H | 3-Heptyl-isoxazol-5-methyl | 2926, 2854, 1606, 1461, 1429, 1388, 1380, 1133 $cm^{-1}$ |
| 296 | H | $CH_3$ | $CH_3$ | $CH_3$ | $CH_3$ | 3-Heptyl-isoxazol-5-methyl | |
| 297 | H | $CH_3$ | $CH_3$ | $CH_3$ | H | 1-Tert.Butyl-pyrrol-3-methyl | |
| 298 | H | $CH_3$ | $CH_3$ | $CH_3$ | $CH_3$ | 1-Tert.Butyl-pyrrol-3-methyl | |
| 301 | H | $CH_3$ | $CH_3$ | $CH_3$ | H | 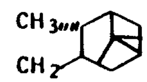 | |
| 302 | H | $CH_3$ | $CH_3$ | $CH_3$ | $CH_3$ | 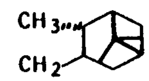 | |
| 303 | H | $CH_3$ | $CH_3$ | $CH_3$ | H | 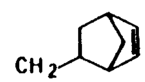 | |
| 304 | H | $CH_3$ | $CH_3$ | $CH_3$ | $CH_3$ | 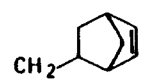 | |
| 305 | H | $CH_3$ | $CH_3$ | $CH_3$ | H | 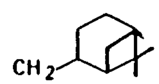 | |

Tabelle 2 (Fortsetzung)

| Verb. Nr. | A | $R^1$ | $R^2$ | $R^3$ | $R^4$ | $R^5$ | Physikalische Daten: Schmp., Sdp. (°C); IR ($cm^{-1}$) |
|---|---|---|---|---|---|---|---|
| 306 | H | $CH_3$ | $CH_3$ | $CH_3$ | $CH_3$ | $CH_2$ 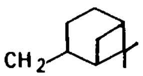 | |
| 307 | H | $CH_3$ | $CH_3$ | $CH_3$ | H | $CH_2-CH_2$ 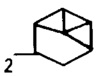 | |
| 308 | H | $CH_3$ | $CH_3$ | $CH_3$ | $CH_3$ | $CH_2-CH_2$ 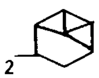 | |
| 309 | OH | H | $CH_3$ | H | H | p-tert.Butyl-benzyl | |
| 310 | OH | H | $CH_3$ | H | $CH_3$ | p-tert.Butyl-benzyl | |
| 311 | OH | H | $CH_3$ | H | H | p-tert.Butyloxy-benzyl | 2972, 2919, 2857, 1508, 1467, 1364, 1239, 1160, 1038, 893 |
| 312 | OH | H | $CH_3$ | H | $CH_3$ | p-tert.Butyloxy-benzyl | |
| 313 | OH | H | $CH_3$ | H | H | p-(1-methoxy-1-methyl-ethyl)-benzyl | |
| 314 | OH | H | $CH_3$ | H | $CH_3$ | p-(1-methoxy-1-methyl-ethyl)-benzyl | |
| 315 | OH | H | $CH_3$ | H | H | p-tert.Butyl-cyclohexyl-methyl | |
| 317 | OH | H | $CH_3$ | H | $CH_3$ | p-tert.Butyl-cyclohexyl-methyl | |
| 318 | OH | H | $CH_3$ | H | H | p-tert.Butyloxy-cyclohexyl-methyl | |
| 319 | OH | H | $CH_3$ | H | H | p-tert.Butyl-benzyl | |
| 320 | OH | H | $CH_3$ | H | $CH_3$ | p-tert.Butyl-benzyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. PolyoxyethylenFettalkohol-Ether,Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 58 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 74 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 47 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser

erhält man eine wäßrige Dispersion.

IV. 20 Gew.-Teile der Verbindung Nr. 243 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 53 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 75 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 84 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 122 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 124 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurden Dekal-2-yl-(2-Methyl-prop-3-yl)-N-cis-2,6-dimethylmorpholin (A) - bekannt aus EP 254 150 - und N,N-Dimethyl-2-amino-dekalin (B) - bekannt aus Liebigs Ann. Chemie, Band 683, Seiten 49 - 54 (1965) verwendet.

Anwendungsbeispiel 2

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea inoculiert und bei 22 - 24 °C in eine Kammer mit hoher Luftfeuchtigkeit gestellt. Nach 5 Tagen hatte sich die Krankheit auf den ubehandelten Kontrollpflanzen so stark entwickelt, daß die entstandenen Blattnekrosen den überwiegenden Teil der Blätter bedeckten.

Das Ergebnis des Versuches zeigt, daß die Wirkstoffe 122, 124, 129, 130, 144, 158, 160 und 311 bei der Anwendung als 0,05 %ige Spritzbrühe eine bessere fungizide Wirkung zeigen (97 %) als die bekannten Vergleichswirkstoffe A und B (35 %).

Anwendungsbeispiel 3

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperononspora) inoculiert. Danach wurden die Reben zunächst für 48 Stunden in einer wasserdampfgesättigten Kammer bei 24 °C und anschließend für 5 Tage in einem Gewächshaus mit Temperaturen

zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigt, daß die Wirkstoffe 120, 122, 123, 126, 129, 130, 132, 141, 142, 166 und 311 bei der Anwendung als 0,05 %iger Spritzbrühe eine bessere fungizide Wirkung zeigen (97 %) als die bekannten Vergleichswirkstoffe A und B (35 %).

**Patentansprüche**

**1.** 2-Aminodekalin-Derivate der Formel I

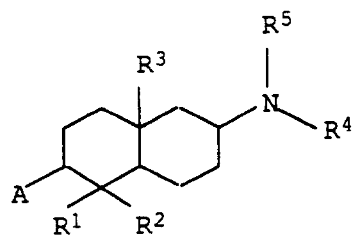

I

in der die Substituenten $R^1$ bis $R^5$ und A die folgende Bedeutung haben:
$R^1$, $R^2$, $R^3$ sind gleich oder verschieden und bedeuten H oder Methyl,
A = OH, O-$C_1$-$C_4$-Alkylcarbonyl, O-Benzoyl,
$R^4$ = H, $C_1$-$C_4$-Alkyl, $C_2$-$C_4$-Alkenyl oder $C_3$-$C_4$-Alkinyl, Cyclopropyl,
$R^5$ = $C_3$-$C_{18}$-Alkyl, $C_3$-$C_{18}$-Alkenyl oder $C_3$-$C_{18}$-Alkinyl, welches gegebenenfalls einfach bis dreifach durch $C_1$-$C_8$-Alkoxy substituiert ist,
$C_5$-$C_{12}$-Cycloalkyl oder $C_5$-$C_{12}$-Cycloalkenyl, welches gegebenenfalls einfach bis dreifach durch $C_3$-$C_8$-Alkyl, $C_3$-$C_8$-Alkenyl oder $C_3$-$C_8$-Alkoxy substituiert ist,
oder $R^5$ bedeutet die Reste

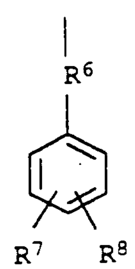

a

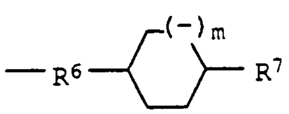

b

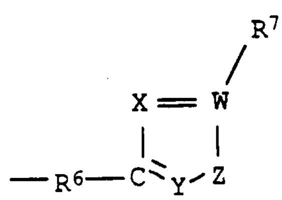

c

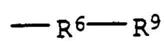

d

worin die Reste $R^6$ bis $R^9$, die Buchstaben W, X, Y, Z sowie der Index m die folgende Bedeutung haben:
$R^6$ = $C_1$-$C_5$-Alkylen oder $C_2$-$C_5$-Alkenylen,
$R^7$ = $C_3$-$C_8$-Alkyl, $C_3$-$C_8$-Alkenyl, $C_3$-$C_8$-Alkoxy, $C_1$-$C_3$-Alkoxy-$C_2$-$C_5$-Alkyl, $C_3$-$C_5$-Alkylthio oder Trimethylsilyl,
$R^8$ = H, $CH_3$,
$R^9$ = gegebenenfalls mit 2 bis 3 Methylgruppen substituiertes $C_6$-$C_{10}$-Bicycloalkyl oder $C_6$-$C_{10}$-Bicycloalkenyl,
W, X, Y, Z = bedeuten die Ringatome in einem aromatischen 5-Ring Heterocyclus, und zwar 1 bis 2 Reste aus der Gruppe O, S, N, NH, N-$R^7$ und 2 bis 3 Reste CH, C-$R^7$,
m bedeuten 0, 1, 2; so daß der Rest 5b einen Cyclohexyl-, Cycloheptyl- oder Cyclopentylrest enthält,
Wenn A O-$C_1$-$C_4$-Alkylcarbonyl oder O-Benzoyl bedeutet, können $R^4$ und $R^5$ auch gemeinsam mit dem Stickstoff, dessen Substituenten sie sind, einen 5- oder 6-gliedrigen Ring e oder f bedeuten,

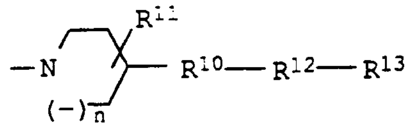 oder 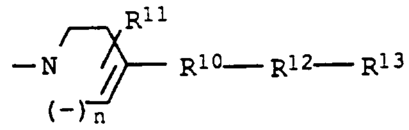

e f

wobei die Reste $R^{10}$ bis $R^{13}$ sowie der Index n die folgende Bedeutung haben:
$R^{10}$ = H oder $(CH_2)_q$ mit q = 0, 1, 2, 3, 4 oder Methyl, Ethyl oder verzweigtes $C_3$-$C_8$-Alkyl,
$R^{11}$ = H, $C_1$-$C_5$-Alkyl,
$R^{12}$, wenn $R^{10}$ verschieden von H ist, bedeutet $R^{12}$ H oder Phenyl,
$R^{13}$, für den Fall, daß $R^{12}$ Phenyl bedeutet, bedeutet $R^{13}$ $C_3$-$C_6$-Alkyl,
n = 0, 1, so daß der Rest 5e oder 5f einen 6-Ring oder 5-Ring Heterocyclus enthält,
zusätzlich bedeutet
A = H, O-$CH_3$, in diesem Falle bedeutet
$R^4$ = H, $C_1$-$C_4$-Alkyl, $C_2$-$C_4$-Alkenyl oder $C_3$-$C_4$-Alkinyl, Cyclopropyl,
$R^5$ bedeutet die Reste c und d

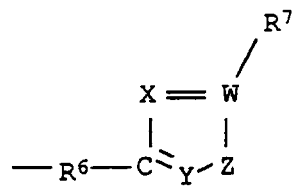 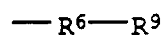

c d

worin die Reste $R^6$ bis $R^9$ und die Buchstaben W, X, Y, Z die folgende Bedeutung haben:
$R^6$ = $C_1$-$C_5$-Alkylen oder $C_2$-$C_5$-Alkenylen,
$R^7$ = $C_3$-$C_8$-Alkyl, $C_3$-$C_8$-Alkenyl, $C_3$-$C_8$-Alkoxy, $C_1$-$C_3$-Alkoxy-$C_2$-$C_5$-Alkyl, $C_3$-$C_5$-Alkylthio oder Trimethylsilyl,
$R^9$ = gegebenenfalls mit 2 bis 3 Methylgruppen substituiertes $C_6$-$C_{10}$-Bicycloalkyl oder $C_6$-$C_{10}$-Bicycloalkenyl,
W, X, Y, Z = bedeuten die Ringatome in einem aromatischen 5-Ring Heterocyclus, und zwar 1 bis 2 Reste aus der Gruppe O, S, N, NH, N-$R^7$ und 2 bis 3 Reste CH, C-$R^7$,
Wenn A H, OH oder O-$CH_3$ bedeuten, können $R^4$ und $R^5$ auch gemeinsam mit dem Stickstoff, dessen Substituenten sie sind, einen 5- oder 6-gliedrigen Ring e bedeuten,

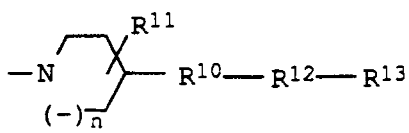

e

wobei die Reste $R^{10}$ bis $R^{13}$ sowie der Index n die folgende Bedeutung haben:
n = 1
$R^{10}$ = $(CH_2)_q$ mit q = 0, 1, 2, 3, 4 oder Ethyl oder verzweigtes $C_3$-$C_8$-Alkyl,
$R^{11}$ = H, $C_1$-$C_5$-Alkyl,
$R^{12}$, wenn $R^{10}$ den Rest $(CH_2)_q$ mit q = 0, 1, 2, 3, 4 bedeutet, bedeutet $R^{12}$ Phenyl und,
$R^{13}$ bedeutet $C_3$-$C_6$-Alkyl,
zusätzlich bedeutet n = 0, in diesem Fall bedeutet $R^{10}$ = $(CH_2)_q$ mit q = 1, 2, 3, 4 oder Ethyl oder verzweigtes $C_3$-$C_8$-Alkyl,

$R^{11}$ = H, $C_1$-$C_5$-Alkyl,
$R^{12}$, wenn $R^{10}$ den Rest $(CH_2)_q$ mit q = 1, 2, 3, 4 bedeutet, bedeutet $R^{12}$ Phenyl und,
$R^{13}$ bedeutet $C_3$-$C_6$-Alkyl,
$R^4$ und $R^5$ können auch gemeinsam mit dem Stickstoff, dessen Substituenten sie sind, einen 5- oder 6-gliedrigen Ring f bedeuten,

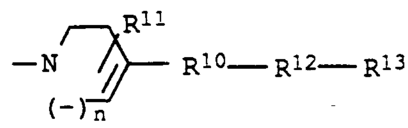

f

wobei die Reste $R^{10}$ bis $R^{13}$ sowie der Index n die folgende Bedeutung haben:
$R^{10}$ = $(CH_2)_q$ mit q = 0, 1, 2, 3, 4 oder Methyl, Ethyl oder verzweigtes $C_3$-$C_8$-Alkyl,
$R^{11}$ = H, $C_1$-$C_5$-Alkyl,
$R^{12}$, wenn $R^{10}$ den Rest $(CH_2)_q$ mit q = 0, 1, 2, 3, 4 bedeutet, bedeutet $R^{12}$ Phenyl, und
$R^{13}$ bedeutet $C_3$-$C_6$-Alkyl,
n = 0, 1, so daß der Rest 5e oder 5f einen 6-Ring oder 5-Ring Heterocyclus enthält,
sowie deren pflanzenverträgliche Salze und N-Oxide.

**2.** 2-Amino-trans-dekalin-Derivate der Formel II

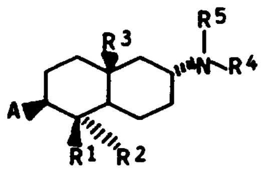

II

in denen ein trans-Dekalin-Gerüst vorliegt, die Substituenten A und $R^3$ cis-ständig, $R^2$ und $NR^4R^5$ trans-ständig sind und die Substituenten $R^1$ - $R^5$ und A dieselben Bedeutungen haben wie in Anspruch 1 sowie deren pflanzenverträgliche Salze und N-Oxide.

**3.** Verfahren zur Herstellung der 2-Amino-dekaline der Formel I gemäß Anspruch 1, dadurch gekennzeichnet, daß man
a) ein Dekalylamin der Formel III ggf. in zwei Stufen alkyliert oder
b) ein $\beta$-Dekalon-Derivat der Formel IV entweder einstufig oder über die Imin-/Iminiumsalz-Stufe V unter Verwendung eines Reduktionsmittels mit einem substituierten Amin VI aminiert,

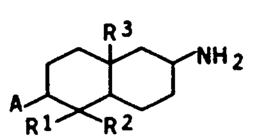

III

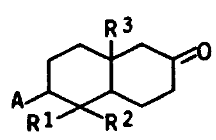

IV

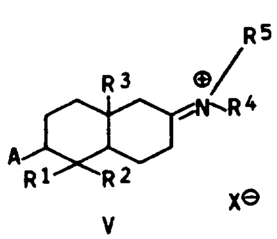

V

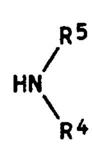

VI

wobei die Reste A und $R^1$ - $R^5$ dieselbe Bedeutung wie in Anspruch I haben und $X^{\ominus}$ ein beliebiges Anion darstellt.

4. Fungizides Mittel, enthaltend eine fungizid wirksame Menge einer Verbindung der Formel I gemäß Anspruch 1, und einen festen oder flüssigen Trägerstoff.

5. Verfahren zur Herstellung von Fungiziden, dadurch gekennzeichnet, daß man eine Verbindung der Formel I gemäß Anspruch 1 mit einem festen oder flüssigen Trägerstoff mischt.

6. Verfahren zur Bekämpfung von Pilzen, dadurch gekennzeichnet, daß man eine fungizid wirksame Menge einer Verbindung der Formel I gemäß Anspruch 1 auf die Pilze oder auf durch Pilzbefall bedrohte Materialien, Flächen, Pflanzen oder Saatgut einwirken läßt.

7. Verbindung der Formel I gemäß Anspruch 1, dadurch gekennzeichnet, daß A, $R^1$, $R^2$ und $R^3$ Wasserstoff und $R^4$ und $R^5$ zusammen mit dem Stickstoffatom, dessen Substituenten sie sind, den 4-tert.Butyl-piperidinylrest bedeuten.

## Claims

1. A 2-aminodecalin derivative of the formula I

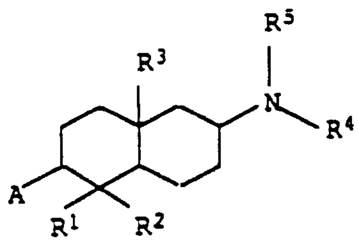

I

where $R^1$, $R^2$ and $R^3$ are identical or different and are each H or methyl,
A is OH, O-$C_1$-$C_4$-alkylcarbonyl or O-benzoyl,
$R^4$ is H, $C_1$-$C_4$-alkyl, $C_2$-$C_4$-alkenyl, $C_3$- or $C_4$-alkynyl or cyclopropyl,
$R^5$ is $C_3$-$C_{18}$-alkyl, $C_3$-$C_{18}$-alkenyl or $C_3$-$C_{18}$-alkynyl which is unsubstituted or monosubstituted to trisubstituted by $C_1$-$C_8$-alkoxy,
$C_5$-$C_{12}$-cycloalkyl or $C_5$-$C_{12}$-cycloalkenyl which is unsubstituted or monosubstituted to trisubstituted by $C_3$-$C_8$-alkyl, $C_3$-$C_8$-alkenyl or $C_3$-$C_8$-alkoxy,
or $R^5$ is a radical

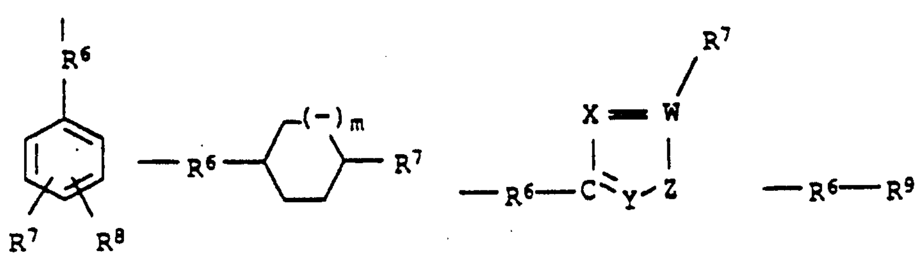

a b c d

where $R^6$ is $C_1$-$C_5$-alkylene or $C_2$-$C_5$-alkenylene,
$R^7$ is $C_3$-$C_8$-alkyl, $C_3$-$C_8$-alkenyl, $C_3$-$C_8$-alkoxy, $C_1$-$C_3$-alkoxy-$C_2$-$C_5$alkyl, $C_3$-$C_5$-alkylthio or trimethylsilyl,
$R^8$ is H or $CH_3$
$R^9$ is $C_6$-$C_{10}$-bicycloalkyl or $C_6$-$C_{10}$-bicycloalkenyl which is unsubstituted or substituted by 2 or 3 methyl groups,

W, X, Y and Z are each a ring atom in an aromatic 5-membered heterocyclic ring, ie. 1 or 2 radicals from the group consisting of O, S, N, NH, N-$R^7$ and 2 or 3 radicals CH and C-$R^7$,
m is 0, 1 or 2, so that the radical 5b contains a cyclohexyl, cycloheptyl or cyclopentyl radical,
when A is O-$C_1$-$C_4$-alkylcarbonyl or O-benzoyl, $R^4$ and $R^5$, together with the nitrogen atom on which they are substituents, may furthermore form a 5-membered or 6-membered ring e or f

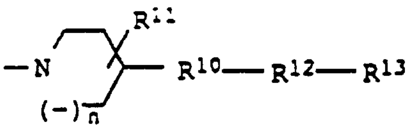

e

or

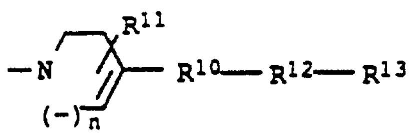

f

where $R^{10}$ is H or $(CH_2)_q$, in which q is 0, 1, 2, 3 or 4 or methyl, ethyl or branched $C_3$-$C_8$-alkyl,
$R^{11}$ is H or $C_1$-$C_5$-alkyl,
$R^{12}$ is H or phenyl when $R^{10}$ is not H,
$R^{13}$ is $C_3$-$C_6$-alkyl when $R^{12}$ is phenyl, and
n is 0 or 1, so that the radical 5e or 5f contains a 6-membered or 5-membered heterocyclic ring,
A is additionally H or O-$CH_3$, in which case
$R^4$ is H, $C_1$-$C_4$-alkyl, $C_2$-$C_4$-alkenyl, $C_3$-$C_4$-alkynyl or cyclopropyl,
$R^5$ is a radical c or d

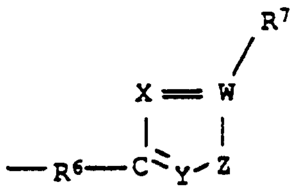

c

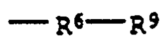

d

where
$R^6$ is $C_1$-$C_5$-alkylene or $C_2$-$C_5$-alkenylene,
$R^7$ is $C_3$-$C_8$-alkyl, $C_3$-$C_8$-alkenyl, $C_3$-$C_8$-alkoxy, $C_1$-$C_3$-alkoxy-$C_2$-$C_5$-alkyl, $C_3$-$C_5$-alkylthio or trimethylsilyl,
$R^9$ is $C_6$-$C_{10}$-bicycloalkyl or $C_6$-$C_{10}$-bicycloalkenyl which is unsubstituted or substituted by 2 or 3 methyl groups,
W, X, Y and Z are each a ring atom in an aromatic 5-membered heterocyclic ring, ie. 1 or 2 radicals from the group consisting of O, S, N, NH, N-$R^7$ and 2 or 3 radicals CH and C-$R^7$,
when A is H, OH or O-$CH_3$, $R^4$ and $R^5$, together with the nitrogen on which they are substituents, may furthermore form a 5-membered or 6-membered ring e

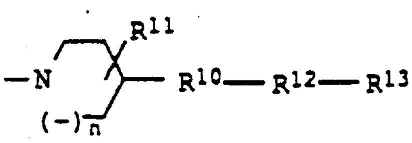

e

where
n is 1,
$R^{10}$ is $(CH_2)_q$, in which q is 0, 1, 2, 3 or 4, or ethyl or branched $C_3$-$C_8$-alkyl,
$R^{11}$ is H or $C_1$-$C_5$-alkyl,

$R^{12}$ is phenyl when $R^{10}$ is $(CH_2)_q$ in which q is 0, 1, 2, 3 or 4, and
$R^{13}$ is $C_3$-$C_6$-alkyl,
n is additionally 0, in which case $R^{10}$ is $(CH_2)_q$, in which q is 1, 2, 3 or 4, or ethyl or branched $C_3$-$C_8$-alkyl,
$R^{11}$ is H or $C_1$-$C_5$-alkyl,
$R^{12}$ is phenyl when $R^{10}$ is $(CH_2)_q$ in which q is 1, 2, 3 or 4,
$R^{13}$ is $C_3$-$C_6$-alkyl,
$R^4$ and $R^5$, together with the nitrogen on which they are substituents, may furthermore form a 5-membered or 6-membered ring f

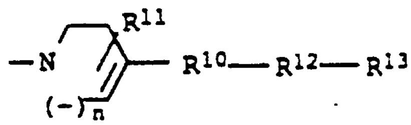

f

where
$R^{10}$ is of $(CH_2)_q$, in which q is 0, 1, 2, 3 or 4, or methyl, ethyl or branched $C_3$-$C_8$-alkyl,
$R^{11}$ is H or $C_1$-$C_5$-alkyl,
$R^{12}$ is phenyl when $R^{10}$ is $(CH_2)_q$ in which q is 0, 1, 2, 3 or 4, and
$R^{13}$ is $C_3$-$C_6$-alkyl and
n is 0 or 1, so that the radical 5e or 5f contains a 6-membered or 5-membered heterocyclic ring,
or a plant-tolerated salt or N-oxide thereof.

2. A 2-amino-trans-decalin derivative of the formula II

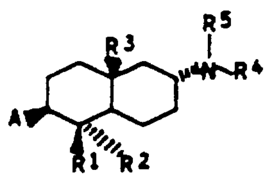

II

which has a trans-decalin skeleton and in which A and $R^3$ are in the cis position,
$R^2$ and $NR^4R^5$ are in the trans position and $R^1$ - $R^5$ and A have the same meanings as in claim 1,
or a plant-tolerated salt or N-oxide thereof.

3. A process for the preparation of a 2-aminodecalin of the formula I as claimed in claim 1, which comprises
   a) alkylating a decalylamine of the formula III, if necessary in two stages, or
   b) aminating a β-decalone derivative of the formula IV, either in a single-stage process or via the imine/iminium salt stage V with the use of a reducing agent, with a substituted amine VI

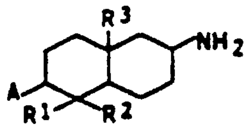

III

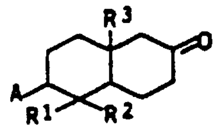

IV

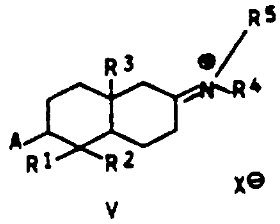

V

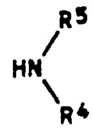

VI

where A and $R^1$ - $R^5$ have the same meanings as in claim 1 and $X^{\ominus}$ is any anion.

**4.** A fungicidal agent containing a fungicidally effective amount of a compound of the formula I as claimed in claim 1, and a solid or liquid carrier.

**5.** A process for the preparation of fungicides, which comprises mixing a compound of the formula I as claimed in claim 1 with a solid or liquid carrier.

**6.** A method for controlling fungi, wherein a fungicidally effective amount of a compound of the formula I as claimed in claim 1 is allowed to act on the fungi or on materials, areas, plants or seed threatened by fungal attack.

**7.** A compound of the formula I as claimed in claim 1, wherein A, $R^1$, $R^2$ and $R^3$ are each hydrogen and $R^4$ and $R^5$, together with the nitrogen atom on which they are substituents, form 4-tert-butylpiperidinyl.

**Revendications**

**1.** Dérivés de 2-aminodécaline de formule I

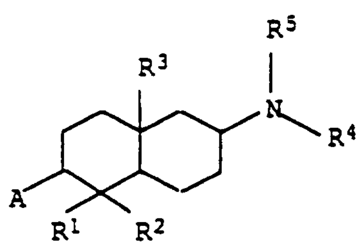

I

dans laquelle les substituants $R^1$ à $R^5$ et A ont les significations suivantes :
$R^1$, $R^2$, $R^3$ sont identiques ou différents et singifient H ou méthyle
A = OH, O-alkyle en C1-C4-carbonyle, O-benzoyle
$R^4$ = H, alkyle en C1-C4, alcényle en C2-C4 ou alcynyle en C3-C4, cyclopropyle
$R^5$ = alkyle en C3-C18, alcényle en C3-C18 ou alcynyle en C3-C18, qui est éventuellement substitué une à trois fois par alcoxy en C1-C8, cycloalkyle en C5-C12, ou cycloalcényle en C5-C12, qui est éventuellement substitué une à trois fois par alkyle en C3-C8, alcényle en C3-C18 ou alcoxy en C3-C8 ou $R^5$ représente les restes

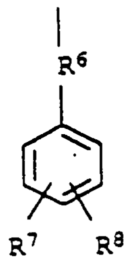 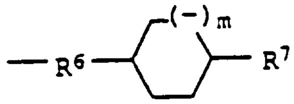 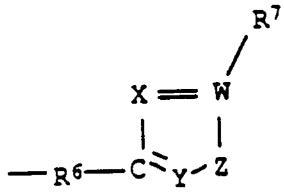 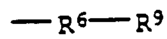

a b c. d

où les restes $R^6$ à $R^9$, les lettres W, X, Y, Z ainsi que l'indice m ont les significations suivantes
$R^6$ = alkylène en C1-C5, ou alcénylène en C2-C5
$R^7$ = alkyle en C3-C8, alcényle en C3-C8, alcoxy en C3-C8, alcoxy en C1-C3 - alkyle en C2-C5, alkyle en C3-C5-thio ou triméthylsilyle
$R^8$ = H, $CH_3$
$R^9$ = bicycloalkyle en C6-C10 ou bicylcoalcényle en C6C-10 substitué éventuellement avec 2 à 3 groupes méthyle
W, X, Y, Z représentent les atomes d'un hétérocycle aromatique à 5 chaînons et ce, 1 à 2 restes des groupes O, S, N, NH, N-$R^7$ et 2 à 3 restes, CH, C-$R^7$
m signifie 0, 1, 2, de sorte que le reste 5b contient un reste cyclohexyle, cycloheptyle cyclopentyle
quand A représente O-(alkyle en C1-C4)-carbonyle ou O-benzyle, $R^4$ et $R^5$ peuvent aussi représenter avec l'azote dont ils sont les substituants, un cycle à 5 ou 6 chaînons e ou f

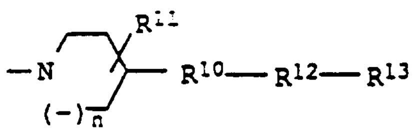 oder 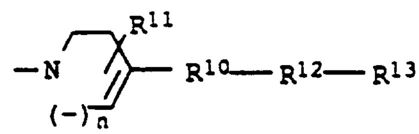

e f

les restes $R^{10}$ à $R^{13}$ ainsi que l'indice n ayant les significations suivantes :
$R^{10}$ = H ou $(CH_2)_q$ avec $_q$ = 0, 1, 2, 3, 4 ou méthyle, éthyle ou alkyle en C3-C8 ramifié
$R^{11}$ = H, alkyle en C1-C5
$R^{12}$, représente, H ou phényle quand $R^{10}$ est différent de H
$R^{13}$, représente alkyle en C3-C6 quand $R^{12}$ représente phényle n = 0,1, de sorte que le reste 5e ou 5f contient un hétérocycle à 6 ou 5 chaînons
en outre, si
A = H, O-$CH_3$, dans ce cas
$R^4$ = H, alkyle en C1-C4, alcényle en C2-C4 ou alcynyle en C3-C4, cyclopropyle
$R^5$ représente les restes c et d

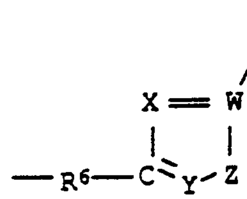 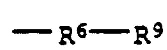

c d

où les restes $R^6$ à $R^9$ et les lettres W, X, Y, Z ont les significations suivantes :
$R^6$ = alkylène en C1-C5 ou alkénylène en C2-C5
$R^7$ = alkyle en C3-C8, alcényle en C3-C8, alcoxy en C3-C8 alcoxy en C1-C3-alkyle en C2-C5, (alkyle

en C3-C5)thio ou triméthylsilyle
$R^9$ = bicycloalkyle en C6-C10 ou bicycloalcényle en C6-C10, éventuellement substitué par 2 à 3 groupes méthyle
W, X, Y, Z = représentent les atomes d'une hétérocycle aromatique à 5 chaînons, et ce, 1 à 2 restes du groupe 0, S, N, NH, N-$R^7$ et 2 à 3 restes CH, C-$R^7$
Quand A représente H, OH ou O-$CH_3$, $R^4$ et $R^5$ peuvent aussi représenter ensemble avec l'azote dont ils sont les substituants, un cycle à 5 ou 6 chaînons e

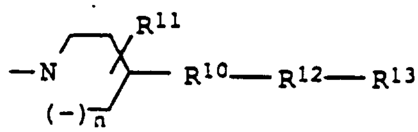

e

les restes $R^{10}$ à $R^{13}$ ainsi que l'indice n ayant les significations suivantes :
n = 1
$R^{10}$ = $(CH_2)_q$ avec q = 0, 1, 2, 3, 4 ou éthyle ou alkyle en C3-C8 ramifié
$R^{11}$ = H, alkyle en C1-C5
$R^{12}$, quand $R^{10}$ signifie le reste $(CH_2)_q$ avec q = 0, 1, 2, 3, 4 R12 représente phényle et
R13 signifie alkyle en C3-C6
en outre n = 0, dans ce cas, $R^{10}$ signifie $(CH_2)_q$ avec q = 1, 2, 3, 4 ou éthyle ou alkyle en C3-C8 ramifié
$R^{11}$ = H, alkyle en C1-C5
$R^{12}$, quand $R^{10}$ signifie le reste $(CH_2)_q$ avec q = 1, 2, 3, 4, $R^{12}$ signifie phényle et
$R^{13}$ signifie alkyle en C3-C6
$R^4$ et $R^5$ peuvent aussi ensemble avec l'azote dont ils sont les substituants, signifier un cycle à 5 ou 6 chaînons f

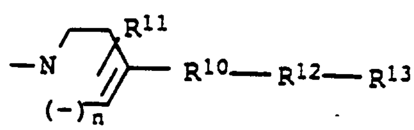

f

les restes $R^{10}$ à $R^{13}$ ainsi que l'indice n ayant les significations suivantes :
$R^{10}$ = $(CH_2)_q$ avec q = 0, 1, 2, 3, 4 ou méthyle, éthyle ou alkyle en C3-C8 ramifié
$R^{11}$ = H, alkyle en C1-C5
$R^{12}$, quand $R^{10}$ signifie le reste $(CH_2)_q$ avec q = 0, 1, 2, 3, 4, $R^{12}$ signifie phényle et
$R^{13}$ signifie alkyle en C3-C6
n = 0, 1, de sorte que le reste 5e ou 5f contient un hétérocycle à 6 ou 5 chaînons
ainsi que leurs sels et N-oxydes acceptables pour les plantes.

**2.** Dérivés de 2-amino-trans-décaline de formule II

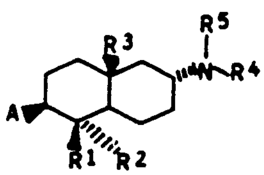

II

dans laquelle existe une structure trans-décaline, les substituants A et $R^3$ sont en position cis, $R^2$ et $NR^4R^5$ en position trans et les substituants $R^1$-$R^5$ et A ont les mêmes significations que dans la

revendication 1
ainsi que leurs sels et oxydes acceptables pour les plantes.

**3.** Procédé de préparation de 2-amino-décaline de formule I selon la revendication 1, caractérisé par le fait que

a) on alkyle, éventuellement en deux phases, une décalylamine de formule III, ou

b) on amine un dérivé de b-décalone de formule IV avec une amine subsituée VI soit en une phase, soit en passant par la phase sel d'imine/iminium (V), en utilisant un agent de réduction

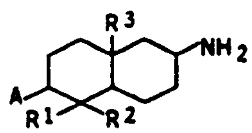

III

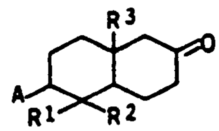

IV

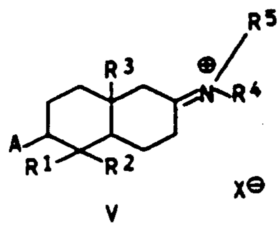

V

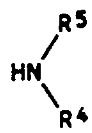

VI

les restes A et $R^1$-$R^5$ ayant la même singification que dans la revendication 1 et $X^{\ominus}$ représentant un anion approprié.

**4.** Fongicide, contenant une quantité efficace du point de vue fongicide d'un composé de formule I selon la revendication 1 et un support solide ou liquide.

**5.** Procédé de préparation de fongicides, caractérisé par le fait que l'on mélange un composé de formule I selon la revendication 1 avec un support solide ou liquide.

**6.** Procédé pour lutter contre les champignons, caractérisé par le fait que l'on fait agir une quantité efficace du point de vue fongicide d'un composé de formule I selon la revendication 1 sur les champignons ou sur les matériaux, surfaces, plantes ou semences menacés d'une attaque par des champignons.

**7.** Composé de formule I selon la revendication 1, caractérisé par le fait que A, $R^{1,}$ $R^2$ et $R^3$ signifient l'hydrogène et $R^4$ et $R^5$ ensemble avec l'atome d'azote dont ils sont les substituants, le reste 4-tert-butylpipéridinyle.